# EUROPEAN PATENT SPECIFICATION

(11) **EP 4 587 143 B1**
(45) Date of publication and mention of the grant of the patent: **09.09.2026**
(21) Application number: 23767912.1
(22) Date of filing: 11.09.2023
(51) Int. Cl.: B01D 15/16, B01D 15/36, B01D 15/42, C12N 15/861, C12N 15/86

(54) **METHOD FOR SEPARATING FULL AND EMPTY AAV PARTICLES**
VERFAHREN ZUR TRENNUNG VON VOLLEN UND LEEREN AAV-PARTIKELN
PROCÉDÉ DE SÉPARATION DE PARTICULES D'AAV PLEINES ET VIDES

(30) Priority: 12.09.2022 EP 22195241
(43) Date of publication of application: 23.07.2025
(60) Divisional application: 26160297.3 / 4 744 752
(73) Proprietor: F. Hoffmann-La Roche AG, 4070 Basel (CH)
(72) Inventor: D'ATRI, Valentina, 1211 Geneve 4 (CH); GUILLARME, Davy, 1211 Geneve 4 (CH); ELGER, Carsten, 82377 Penzberg (DE); KOCHARDT, Dominik, 82377 Penzberg (DE); RUPPERT, Raphael, 82377 Penzberg (DE)
(74) Representative: Kallenbach, Lorenz
(86) International application number: PCT/EP2023/074823
(87) International publication number: WO 2024/056561

(56) References cited:
- WO-A1-2021/158915
- US-A1- 2021 009 964
- US-A1- 2021 355 503
- DICKERSON RYAN ET AL, vol. 16, no. 1, 19 October 2020 (2020-10-19), DE, pages 2000015, XP055802893, ISSN: 1860-6768, Retrieved from the Internet <URL:https://onlinelibrary.wiley.com/doi/full-xml/10.1002/biot.202000015> [retrieved on 20230206], DOI: 10.1002/biot.202000015
- KHATWANI SANTOSHKUMAR ET AL: "Anion-exchange HPLC assay for separation and quantification of empty and full capsids in multiple adeno-associated virus serotypes", 11 June 2021 (2021-06-11), XP093020887, Retrieved from the Internet <URL:http://www.ncbi.nlm.nih.gov/pubmed/33997103> [retrieved on 20230205]

## Description

The current invention is in the field of analytical method. In more detail, herein is reported a method for separating full and empty recombinant AAV particles using a method comprising an isocratic hold.

### Background of the Invention

Gene therapy is opening unprecedented opportunities for novel therapeutic approaches. Based on the concept of rescuing function mutations by co-expressing the correct gene, to allow biological functions to be restored, it requires the use of viral vectors to ensure the proper delivery of therapeutic genes. In this context, recombinant adeno-associated viruses (rAAV) are the most widely used vectors. Their bio manufacturing process requires the insertion of the therapeutic gene into the rAAV (full AAV particles, i.e. rAAV particles comprising an encapsidated nucleic acid). However, a percentage of rAAV that do not contain the desired gene (empty AAV particles, i.e. rAAV particles not comprising an encapsidated nucleic acid) might also be produced, as well as partly filled rAAVs (partly filled AAV particles), potentially impacting the efficiency of the therapy. Therefore, the determination of rAAV particle full/empty ratio needs to be monitored to ensure consistent product quality and efficacy.

Various analytical strategies have been suggested to characterize empty rAAV particle content, including electron microscopy, quantitative polymerase chain reaction (PCR), Elisa assay, UV absorbance spectrophotometry, analytical ultracentrifugation or charge detection mass spectrometry (CDMS) (Gimpel, A.L., et al., Mol. Ther. - Methods Clin. Dev. 20 (2021) 740-754; Fu, X., et al., Hum Gene Ther Methods. 30 (2019) 144-152).

Generally, a difference in the range of 0.4 pH units can be observed between full rAAV particles and empty rAAV particles. This feature might be exploited in the attempt to separate and quantify full and empty rAAV particles by anion exchange chromatography (AEX) (Venkatakrishnan, B., et al., J. Virol. 87 (2013) 4974-4984; Khatwani, S.L., et al., Mol. Ther. - Methods Clin. Dev. 21 (2021) 548-558; Urabe, M., et al., Mol. Ther. 13 (2006) 823-828; Dickerson, R., et al., Biotechnol. J. 16 (2021) 2000015; Kaludov, N., et al., Hum. Gene Ther. 13 (2002) 1235-1243; Lock, M, et al., Hum. Gene Ther. Methods. 23 (2012) 56-64).

In AEX the rAAV particles follow an "on/off" retention behavior (Snyder, L.R., et al., Anal. Chem. 55 (1983) 1412A-1430A; Fekete, S., et al., Anal. Chem. 93 (2021) 1277-1284).

WO 2021/158915 discloses recombinant adeno-associated virus compositions and methods for producing and using the same.

US 2021/355503 discloses compositions and methods for manufacturing gene therapy vectors.

Khatwani Santoshkumar et al. disclose an anion-exchange HPLC assay for separation and quantification of empty and full capsids in multiple adeno-associated virus serotypes (Mol. Ther. Meth. Clin. Dev. 21 (2021) 548-558).

US 2021/009964 discloses the separation and quantification of empty and full viral capsid particles.

Dickerson, R., et al. reported the separation of empty and full recombinant adeno-associated virus particles using isocratic anion exchange chromatography (Biotechnol J 16 (2021) 2000015.

### Summary of the Invention

Herein is reported an anion-exchange chromatography (AEX) method comprising a novel elution scheme. Without being bound by this theory, it is assumed that the method takes advantage of the fact that viral capsids follow an "on/off" retention behavior. Based thereon an elution method has been found that allows the unprecedented separation of full rAAV particles from empty rAAV particles. The advantages of the method according to the invention is, e.g., a resolution gain of 3.7 as compared to the resolution obtainable with a linear gradient. With the method according to the current invention a precise and accurate baseline separation and quantification of full and empty rAAV particles is possible, allowing the application amongst others, e.g., as quality control (QC) method.

The invention is as defined in the claims. In addition, the following embodiments are disclosed:
1. A method for separating full and empty recombinant adeno-associated virus particles using an anion exchange chromatography (AEX) step, wherein the method comprises the following steps:
   a) applying a solution comprising a recombinant AAV particle without encapsidated nucleic acid (empty rAAV particle) and a recombinant AAV particle with encapsidated nucleic acid (full rAAV particle) to an chromatography column comprising an anion exchange (AEX) chromatography material to produce a recombinant AAV particle loaded AEX chromatography column (rAAV-loaded AEX chromatography column), wherein the solution comprising an empty rAAV particle and a full rAAV particle is a phosphate buffered saline solution comprising about 0.001% (w/v) of a non-ionic detergent,
   b) applying a first buffered solution to the rAAV-loaded AEX chromatography column, whereby the rAAV particles remain bound to the AEX chromatography material, to obtain a washed rAAV-loaded AEX chromatography column, wherein the first buffered solution has a first pH value, a first conductivity and comprises a buffer substance, optionally an elution salt and optionally a salt,
   c) applying a first intermediate buffered solution with increasing conductivity starting at the first conductivity and ending at a second conductivity to the washed rAAV-loaded AEX chromatography column to obtain an intermediate-conditioned rAAV-loaded AEX chromatography column, wherein the increase in conductivity is linear and by the addition of the elution salt with increasing concentration,
   d) applying a second buffered solution to the intermediate-conditioned rAAV-loaded AEX chromatography column to obtain a semi-eluted rAAV-loaded AEX chromatography column, wherein the second buffered solution has a second pH value, the second conductivity and comprises the buffer substance, the elution salt and optionally the salt,
   e) applying a second intermediate buffered solution with increasing conductivity starting at the second conductivity and ending at a third conductivity to the semi-eluted rAAV-loaded AEX chromatography column to obtain an eluted AEX chromatography column, wherein the increase in conductivity is linear and by the addition of the elution salt with increasing concentration,

   wherein the second buffered solution has a conductivity that is increased with respect to the first buffered solution by about 8 mS/cm to 9.5 mS/cm
   wherein in step c) the empty rAAV particle is eluted,
   wherein the applying in step d) is for at least 2 minutes,
   wherein in step e) the full rAAV particle is eluted,
   wherein the conductivity is increased in step e) by at least about 20 mS/cm.
2. The method according to embodiment 1, wherein the rAAV particles are of the serotype 8.
3. The method according to any one of embodiments 1 to 2, wherein the anion exchange chromatography is a strong anion exchange chromatography.
4. The method according to any one of embodiments 1 to 3, wherein the anion exchange chromatography material is a stationary phase consisting of microporous ethylvinylbenzene cross-linked with 55 % divinylbenzene polymer substrate with quaternary ammonium groups providing for the anion exchange functionality.
5. The method according to any one of embodiments 1 to 4, wherein the AEX chromatography column has the dimension of about 50 mm length and about 4-5 mm diameter.
6. The method according to any one of embodiments 1 to 5, wherein the AEX chromatography material has a particle size of about 10 µm.
7. The method according to any one of embodiments 1 to 6, wherein the method is for/provides baseline separation of full and empty rAAV particles.
8. The method according to any one of embodiments 1 to 7, wherein the method is for quantification of full and empty rAAV particles.
9. The method according to any one of embodiments 1 to 8, wherein the method is a high-throughput method for quantification of full and empty rAAV particles rAAV.
10. The method according to any one of embodiments 1 to 9, wherein the method is a quality control (QC) method for rAAV preparations.
11. The method according to any one of embodiments 1 to 10, wherein the solution comprising an empty rAAV particle and a full rAAV particle is a phosphate buffered saline solution (comprising 137 mM NaCl, 2.7 mM KCl, 10 mM Na₂HPO₄, 1.8 mM KH₂PO₄) comprising about 0.001 % (w/v) of a non-ionic detergent.
12. The method according to any one of embodiments 1 to 11, wherein the solution comprising an empty rAAV particle and a full rAAV particle, is a buffered solution comprising about 0.001 % (w/v) of poloxamer 188 or polysorbate 20.
13. The method according to any one of embodiments 1 to 12, wherein the solution comprising an empty rAAV particle and a full rAAV particle is a phosphate buffered saline solution (comprising 137 mM NaCl, 2.7 mM KCl, 10 mM Na₂HPO₄, 1.8 mM KH₂PO₄) comprising about 0.001 % (w/v) of poloxamer 188 or polysorbate 20.
14. The method according to any one of embodiments 1 to 13, wherein the first and second buffered solution as well as the first and the second intermediate buffered solutions comprise a buffer substance with a pKa value of about 9.
15. The method according to any one of embodiments 1 to 14, wherein the first and second buffered solution as well as the first and the second intermediate buffered solutions comprise a buffer substance selected from 1,3-Bis[tris(hydroxymethyl)methylamino]propane and N-(1,1-Dimethyl-2-hydroxyethyl)-3-amino-2-hydroxypropane sulfonic acid.
16. The method according to any one of embodiments 1 to 15, wherein the first and second buffered solution as well as the first and the second intermediate buffered solutions comprise have a pH value of 9.0 to 10.0.
17. The method according to any one of embodiments 1 to 16, wherein the first and second buffered solution as well as the first and the second intermediate buffered solutions comprise have a pH value of 9.2 to 9.6.
18. The method according to any one of embodiments 1 to 17, wherein the first and second buffered solution as well as the first and the second intermediate buffered solutions comprise have a pH value of about 9.4.
19. The method according to any one of embodiments 1 to 18, wherein the first and second buffered solution as well as the first and the second intermediate buffered solutions comprise an organic salt as elution salt.
20. The method according to any one of embodiments 1 to 19, wherein the first and second buffered solution as well as the first and the second intermediate buffered solutions comprise an organic chloride as elution salt.
21. The method according to any one of embodiments 1 to 20, wherein the first and second buffered solution as well as the first and the second intermediate buffered solutions comprise an organic chloride with a molecular weight below 170 g/mol as elution salt.
22. The method according to any one of embodiments 1 to 21, wherein the first and second buffered solution as well as the first and the second intermediate buffered solutions comprise tetraethyl ammonium chloride as elution salt.
23. The method according to any one of embodiments 1 to 21, wherein the first and second buffered solution as well as the first and the second intermediate buffered solutions comprise tetramethyl ammonium chloride as elution salt.
24. The method according to any one of embodiments 1 to 22, wherein the method is performed at a flow rate of about 0.3 mL/min.
25. The method according to any one of embodiments 1 to 24, wherein the first buffered solution comprises about 65 mM of the buffer substance, about 10 mM of the elution salt, about 2 mM of the salt and has a pH value of about 9.4.
26. The method according to any one of embodiments 1 to 25, wherein the second buffered solution comprises about 65 mM of the buffer substance, about 90 mM of the elution salt, about 2 mM of the salt and has a pH value of about 9.4.
27. The method according to any one of embodiments 1 to 26, wherein the buffer substance is N-(1,1-Dimethyl-2-hydroxyethyl)-3-amino-2-hydroxypropane sulfonic acid, the elution salt is tetramethyl ammonium chloride, and the salt is magnesium chloride.
28. The method according to any one of embodiments 1 to 27, wherein step b) has a length of about 1 minute, step c) has a length of about 5 minutes, step d) has a length of at least 2 minutes and at most 5 minutes, and step e) has a length of about 5 minutes.
29. A method for separating full and empty recombinant adeno-associated virus particles using an anion exchange chromatography step,
   wherein the method comprises a sequence of a applying a solution comprising empty and full rAAV particles to an anion exchange chromatography material inside a chromatography column, a first isocratic step, a first linear gradient, a second isocratic step and a second linear gradient,
   wherein the empty recombinant adeno-associated virus particles are eluted during the first linear gradient and the full recombinant adeno-associated virus particles are eluted during the second linear gradient,
   wherein
      i) the solution comprising empty and full rAAV particles is a buffered solution comprising about 0.001 % (w/v) of a non-ionic detergent, and
      ii) the rAAV particle is of the serotype 8, or/and
      iii) the anion exchange chromatography material is a strong anion exchange material, or/and
      iv) the anion exchange chromatography material has a stationary phase consisting of microporous ethylvinylbenzene cross-linked with 55 % divinylbenzene polymer substrate with quaternary ammonium groups providing for the anion exchange functionality, or/and
      v) the AEX chromatography column has the dimension of about 50 mm length and about 4-5 mm diameter, or/and
      vi) the AEX chromatography material has a particle size of about 10 µm, or/and
      vii) the method is for/provides baseline separation of full and empty rAAV particles, or/and
      viii) the method is for quantification of full and empty rAAV particles, or/and
      ix) the method is a high-throughput method for quantification of full and empty rAAV particles rAAV, or/and
      x) the method is a quality control (QC) method for rAAV preparations, or/and
      xi) the solution comprising empty and/or full rAAV particles is a phosphate buffered saline solution (comprising 137 mM NaCl, 2.7 mM KCl, 10 mM Na₂HPO₄, 1.8 mM KH₂PO₄) comprising about 0.001 % (w/v) of poloxamer 188 or polysorbate 20, or/and
      xii) the solutions used in the isocratic steps and linear gradients comprise a buffer substance with a pKa value of about 9, or/and
      xiii) the solutions used in the isocratic steps and linear gradients comprise a buffer substance selected from 1,3-Bis[tris(hydroxymethyl)methylamino]propane and N-(1,1-Dimethyl-2-hydroxyethyl)-3-amino-2-hydroxypropane sulfonic acid, or/and
      xiv) the solutions used in the isocratic steps and linear gradients have a pH value of 9.0 to 10.0, or/and
      xv) the solutions used in the isocratic steps and linear gradients have a pH value of 9.2 to 9.6, or/and
      xvi) the solutions used in the isocratic steps and linear gradients have a pH value of about 9.4, or/and
      xvii) the solutions used in the isocratic steps and linear gradients comprise an organic salt, or/and
      xviii) the solutions used in the isocratic steps and linear gradients comprise an organic chloride as salt, or/and
      xix) the solutions used in the isocratic steps and linear gradients comprise an organic chloride with a molecular weight below 170 g/mol, or/and
      xx) the solutions used in the isocratic steps and linear gradients comprise tetraethyl ammonium chloride, or/and
      xxi) the solutions used in the isocratic steps and linear gradients comprise tetramethyl ammonium chloride, or/and
      xxii) the method is performed at a flow rate of about 0.3 mL/min, or/and
      xxiii) the solution applied in the first isocratic step comprises about 65 mM of the buffer substance, about 10 mM of the elution salt, about 2 mM of the salt and has a pH value of about 9.4, or/and
      xxiv) the solution applied in the second isocratic step comprises about 65 mM of the buffer substance, about 90 mM of the elution salt, about 2 mM of the salt and has a pH value of about 9.4, or/and
      xxv) the buffer substance is N-(1,1-Dimethyl-2-hydroxyethyl)-3-amino-2-hydroxypropane sulfonic acid, the elution salt is tetramethyl ammonium chloride, and the salt is magnesium chloride, or/and
      xxvi) a first isocratic step has a length of about 1 minute, the first linear gradient has a length of about 5 minute, a second isocratic step has a length of about 2 to 5 minutes and the second linear gradient has a length of about 5 minutes.
30. The method according to embodiment 29 comprising items ii), iiii), xii), ix), xvii), xxii), xxiii), xxv), xxvi), or/and xxvii).
31. The method according to embodiment 29 comprising items ii), iv), xii), xiv), xvii), xxii), xxiii), xxv), xxvi), and xxvii).
32. A method for separating full and empty recombinant adeno-associated virus particles using an anion exchange chromatography step,
   wherein the method comprises a sequence of a applying a solution comprising empty and full rAAV particles to an anion exchange chromatography material inside a chromatography column, a first isocratic step, a first linear gradient, a second isocratic step and a second linear gradient,
   wherein the empty recombinant adeno-associated virus particles are eluted during the first linear gradient and the full recombinant adeno-associated virus particles are eluted during the second linear gradient,
   wherein
      x) the solution comprising empty and full rAAV particles is a buffered solution comprising about 0.001 % (w/v) of a non-ionic detergent, and
      i) the rAAV particle is of the serotype 8, or/and
      iii) the anion exchange chromatography material has a stationary phase consisting of microporous ethylvinylbenzene cross-linked with 55 % divinylbenzene polymer substrate with quaternary ammonium groups providing for the anion exchange functionality, or/and
      iv) the AEX chromatography column has the dimension of about 50 mm length and about 4-5 mm diameter, or/and
      v) the AEX chromatography material has a particle size of about 10 µm, or/and
      xi) the solution comprising empty and/or full rAAV particles is a phosphate buffered saline solution (comprising 137 mM NaCl, 2.7 mM KCl, 10 mM Na₂HPO₄, 1.8 mM KH₂PO₄) comprising about 0.001 % (w/v) of poloxamer 188 or polysorbate 20, or/and
      xii) the solutions used in the isocratic steps and linear gradients comprise a buffer substance with a pKa value of about 9, or/and
      xiii) the solutions used in the isocratic steps and linear gradients comprise a buffer substance selected from 1,3-Bis[tris(hydroxymethyl)methylamino]propane and N-(1,1-Dimethyl-2-hydroxyethyl)-3-amino-2-hydroxypropane sulfonic acid, or/and
      xiv) the solutions used in the isocratic steps and linear gradients have a pH value of 9.0 to 10.0, or/and
      xv) the solutions used in the isocratic steps and linear gradients have a pH value of 9.2 to 9.6, or/and
      xvi) the solutions used in the isocratic steps and linear gradients have a pH value of about 9.4, or/and
      xix) the solutions used in the isocratic steps and linear gradients comprise an organic chloride with a molecular weight below 170 g/mol, or/and
      xx) the solutions used in the isocratic steps and linear gradients comprise tetraethyl ammonium chloride, or/and
      xxi) the solutions used in the isocratic steps and linear gradients comprise tetramethyl ammonium chloride, or/and
      xxii) the method is performed at a flow rate of about 0.3 mL/min, or/and
      xxiii) the solution applied in the first isocratic step comprises about 65 mM of the buffer substance, about 10 mM of the elution salt, about 2 mM of the salt and has a pH value of about 9.4, or/and
      xxiv) the solution applied in the second isocratic step comprises about 65 mM of the buffer substance, about 90 mM of the elution salt, about 2 mM of the salt and has a pH value of about 9.4, or/and
      xxv) the buffer substance is N-(1,1-Dimethyl-2-hydroxyethyl)-3-amino-2-hydroxypropane sulfonic acid, the elution salt is tetramethyl ammonium chloride, and the salt is magnesium chloride, or/and
      xxvi) a first isocratic step has a length of about 1 minute, the first linear gradient has a length of about 5 minute, a second isocratic step has a length of about 2 to 5 minutes and the second linear gradient has a length of about 5 minutes.
33. A method for separating full and empty recombinant adeno-associated virus particles using an anion exchange chromatography step,
   wherein the method comprises a sequence of a applying a solution comprising empty and full rAAV particles to an anion exchange chromatography material inside a chromatography column, a first isocratic step, a first linear gradient, a second isocratic step and a second linear gradient,
   wherein the empty recombinant adeno-associated virus particles are eluted during the first linear gradient and the full recombinant adeno-associated virus particles are eluted during the second linear gradient,
   wherein
      x) the solution comprising empty and full rAAV particles is a buffered solution comprising about 0.001 % (w/v) of a non-ionic detergent, and
      i) the rAAV particle is of the serotype 8, or/and
      iii) the anion exchange chromatography material has a stationary phase consisting of microporous ethylvinylbenzene cross-linked with 55 % divinylbenzene polymer substrate with quaternary ammonium groups providing for the anion exchange functionality, or/and
      iv) the AEX chromatography column has the dimension of about 50 mm length and about 4-5 mm diameter, or/and
      v) the AEX chromatography material has a particle size of about 10 µm, or/and
      xi) the solution comprising empty and/or full rAAV particles is a phosphate buffered saline solution (comprising 137 mM NaCl, 2.7 mM KCl, 10 mM Na₂HPO₄, 1.8 mM KH₂PO₄) comprising about 0.001 % (w/v) of poloxamer 188 or polysorbate 20, or/and
      xiii) the solutions used in the isocratic steps and linear gradients comprise a buffer substance selected from 1,3-Bis[tris(hydroxymethyl)methylamino]propane and N-(1,1-Dimethyl-2-hydroxyethyl)-3-amino-2-hydroxypropane sulfonic acid, or/and
      xiv) the solutions used in the isocratic steps and linear gradients have a pH value of 9.0 to 10.0, or/and
      xv) the solutions used in the isocratic steps and linear gradients have a pH value of 9.2 to 9.6, or/and
      xvi) the solutions used in the isocratic steps and linear gradients have a pH value of about 9.4, or/and
      xix) the solutions used in the isocratic steps and linear gradients comprise an organic chloride with a molecular weight below 170 g/mol, or/and
      xxi) the solutions used in the isocratic steps and linear gradients comprise tetramethyl ammonium chloride, or/and
      xxii) the method is performed at a flow rate of about 0.3 mL/min, or/and
      xxiii) the solution applied in the first isocratic step comprises about 65 mM of the buffer substance, about 10 mM of the elution salt, about 2 mM of the salt and has a pH value of about 9.4, or/and
      xxiv) the solution applied in the second isocratic step comprises about 65 mM of the buffer substance, about 90 mM of the elution salt, about 2 mM of the salt and has a pH value of about 9.4, or/and
      xxv) the buffer substance is N-(1,1-Dimethyl-2-hydroxyethyl)-3-amino-2-hydroxypropane sulfonic acid, the elution salt is tetramethyl ammonium chloride, and the salt is magnesium chloride, or/and
      xxvi) a first isocratic step has a length of about 1 minute, the first linear gradient has a length of about 5 minute, a second isocratic step has a length of about 2 to 5 minutes and the second linear gradient has a length of about 5 minutes.
34. A method for separating full and empty recombinant adeno-associated virus particles using an anion exchange chromatography step,
   wherein the method comprises a sequence of a applying a solution comprising empty and full rAAV particles to an anion exchange chromatography material inside a chromatography column, a first isocratic step, a first linear gradient, a second isocratic step and a second linear gradient,
   wherein the empty recombinant adeno-associated virus particles are eluted during the first linear gradient and the full recombinant adeno-associated virus particles are eluted during the second linear gradient,
   wherein
      x) the solution comprising empty and full rAAV particles is a buffered solution comprising about 0.001 % (w/v) of a non-ionic detergent, and
      i) the rAAV particle is of the serotype 8, or/and
      iii) the anion exchange chromatography material has a stationary phase consisting of microporous ethylvinylbenzene cross-linked with 55 % divinylbenzene polymer substrate with quaternary ammonium groups providing for the anion exchange functionality, or/and
      iv) the AEX chromatography column has the dimension of about 50 mm length and about 4-5 mm diameter, or/and
      v) the AEX chromatography material has a particle size of about 10 µm, or/and
      xi) the solution comprising empty and/or full rAAV particles is a phosphate buffered saline solution (comprising 137 mM NaCl, 2.7 mM KCl, 10 mM Na₂HPO₄, 1.8 mM KH₂PO₄) comprising about 0.001 % (w/v) of poloxamer 188, or/and
      xiii) the solutions used in the isocratic steps and linear gradients comprise as buffer substance N-(1,1-Dimethyl-2-hydroxyethyl)-3-amino-2-hydroxypropane sulfonic acid, or/and
      xiv) the solutions used in the isocratic steps and linear gradients have a pH value of 9.0 to 10.0, or/and
      xv) the solutions used in the isocratic steps and linear gradients have a pH value of 9.2 to 9.6, or/and
      xvi) the solutions used in the isocratic steps and linear gradients have a pH value of about 9.4, or/and
      xxi) the solutions used in the isocratic steps and linear gradients comprise tetramethyl ammonium chloride, or/and
      xxii) the method is performed at a flow rate of about 0.3 mL/min, or/and
      xxiii) the solution applied in the first isocratic step comprises about 65 mM of the buffer substance, about 10 mM of the elution salt, about 2 mM of the salt and has a pH value of about 9.4, or/and
      xxiv) the solution applied in the second isocratic step comprises about 65 mM of the buffer substance, about 90 mM of the elution salt, about 2 mM of the salt and has a pH value of about 9.4, or/and
      xxv) the buffer substance is N-(1,1-Dimethyl-2-hydroxyethyl)-3-amino-2-hydroxypropane sulfonic acid, the elution salt is tetramethyl ammonium chloride, and the salt is magnesium chloride, or/and
      xxvi) a first isocratic step has a length of about 1 minute, the first linear gradient has a length of about 5 minute, a second isocratic step has a length of about 2 to 5 minutes and the second linear gradient has a length of about 5 minutes.
35. A method for separating full and empty recombinant adeno-associated virus particles using an anion exchange chromatography step,
   wherein the method comprises a sequence of a applying a solution comprising empty and full rAAV particles to an anion exchange chromatography material inside a chromatography column, a first isocratic step, a first linear gradient, a second isocratic step and a second linear gradient,
   wherein the empty recombinant adeno-associated virus particles are eluted during the first linear gradient and the full recombinant adeno-associated virus particles are eluted during the second linear gradient,
   wherein the rAAV particle is of the serotype 8, and
   wherein the solution comprising empty and full rAAV particles is a buffered solution comprising about 0.001 % (w/v) of a non-ionic detergent, or
   wherein
   the solution comprising empty and full rAAV particles is a buffered solution comprising about 0.001 % (w/v) of a non-ionic detergent, and
   i) the anion exchange chromatography material has a stationary phase consisting of microporous ethylvinylbenzene cross-linked with 55 % divinylbenzene polymer substrate with quaternary ammonium groups providing for the anion exchange functionality, and
   ii) the AEX chromatography column has the dimension of about 50 mm length and about 4-5 mm diameter, and
   iii) the AEX chromatography material has a particle size of about 10 µm, and
   iv) the solution comprising empty and/or full rAAV particles is a phosphate buffered saline solution (comprising 137 mM NaCl, 2.7 mM KCl, 10 mM Na₂HPO₄, 1.8 mM KH₂PO₄) comprising about 0.001 % (w/v) of poloxamer 188, and
   v) the solutions used in the isocratic steps and linear gradients comprise as buffer substance N-(1,1-Dimethyl-2-hydroxyethyl)-3-amino-2-hydroxypropane sulfonic acid, and
   vi) the solutions used in the isocratic steps and linear gradients have a pH value of about 9.4, and
   vii) the solutions used in the isocratic steps and linear gradients comprise tetramethyl ammonium chloride, and
   viii) the method is performed at a flow rate of about 0.3 mL/min, and
   ix) the solution applied in the first isocratic step comprises about 65 mM of the buffer substance, about 10 mM of the elution salt, about 2 mM of the salt and has a pH value of about 9.4, and
   x) the solution applied in the second isocratic step comprises about 65 mM of the buffer substance, about 90 mM of the elution salt, about 2 mM of the salt and has a pH value of about 9.4, and
   xi) the buffer substance is N-(1,1-Dimethyl-2-hydroxyethyl)-3-amino-2-hydroxypropane sulfonic acid, the elution salt is tetramethyl ammonium chloride, and the salt is magnesium chloride, and
   xii) the first isocratic step has a length of about 1 minute, the first linear gradient has a length of about 5 minute, a second isocratic step has a length of about 2 to 5 minutes and the second linear gradient has a length of about 5 minutes.

In addition to the various embodiments depicted and claimed, the disclosed subject matter is also directed to other embodiments having other combinations of the features disclosed and claimed herein. As such, the particular features presented herein can be combined with each other in other manners within the scope of the disclosed subject matter such that the disclosed subject matter includes any suitable combination of the features disclosed herein. The foregoing description of specific embodiments of the disclosed subject matter has been presented for purposes of illustration and description. It is not intended to be exhaustive or to limit the disclosed subject matter to those embodiments disclosed.

### Description of the Figures

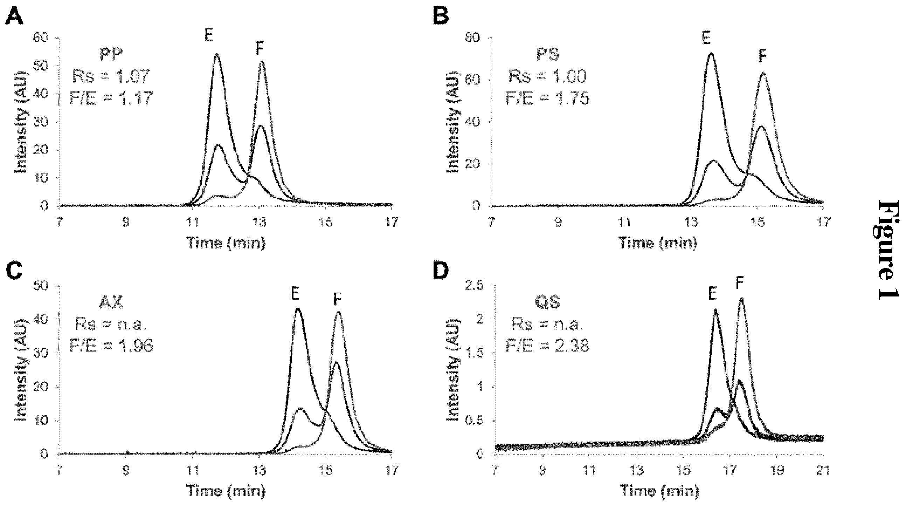

**Figure 1****.** Empty (red line), full (blue line) and mixture of full and empty (violet line) rAAV8 particles were analyzed using 4 different column hardware, namely PP (A), PS (B), AX (C), and QS (D) with a method according to Example 2; E = empty rAAV8 particle, F = full rAAV8 particle, Rs = peak resolution, F/E = full/empty rAAV particle ratio.

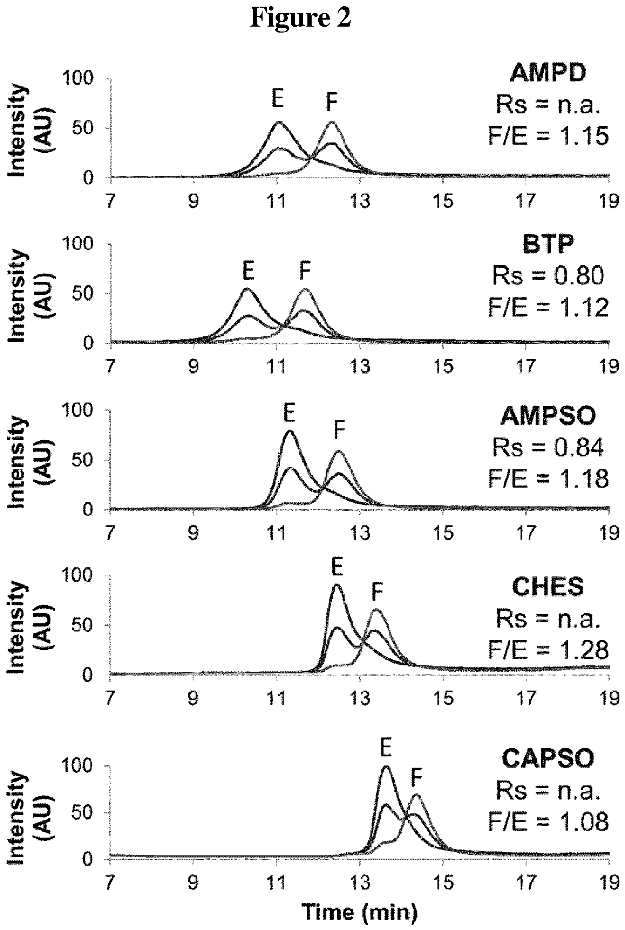

**Figure 2****.** Results with different buffer types. Empty (red line), full (blue line) and mix of full and empty (violet line) rAAV8 particles analyzed in AEX mode by using five different buffer types, namely AMPD, BTP, AMPSO, CHES, and CAPSO with an elution method according to Example 2; E = empty rAAV8 particle, F = full rAAV8 particle, Rs = peak resolution, F/E = full/empty ratio, n.a. = not available.

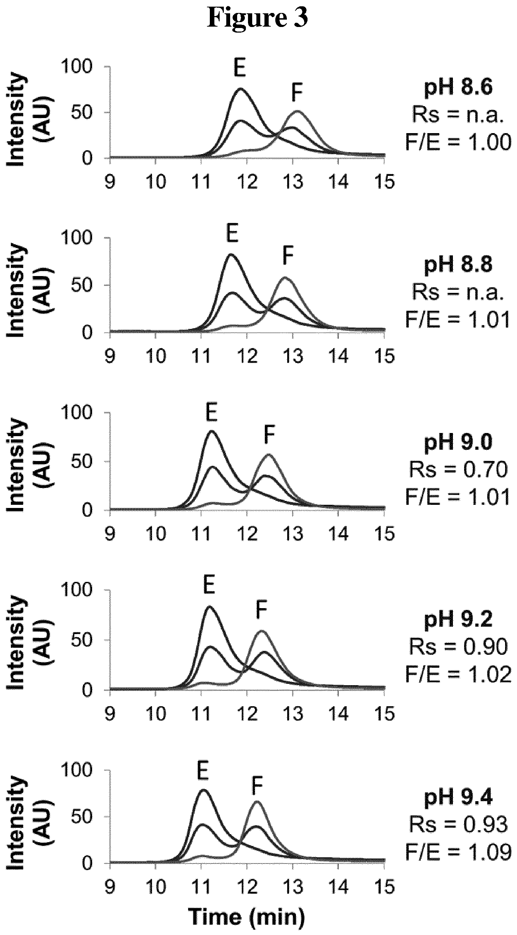

**Figure 3****.** Results with AMPSO buffer at different pH values. Empty (red line), full (blue line) and mix of full and empty (violet line) rAAV8 particles were analyzed in AEX mode at five different pH, namely 8.6, 8.8, 9.0, 9.2, and 9.4 with an elution method according to Example 2; E = empty rAAV8 particle, F = full rAAV8 particle, Rs = peak resolution, F/E = full/empty ratio, n.a. = not available.

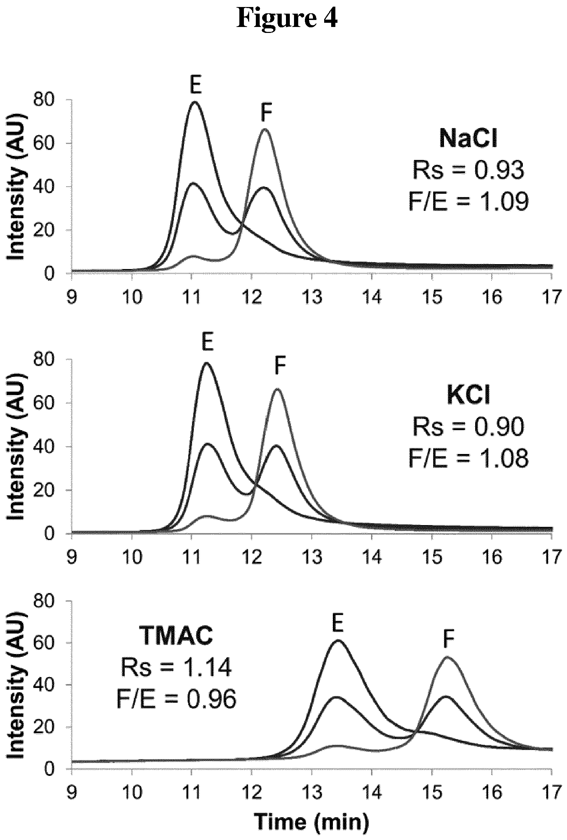

**Figure 4****.** Results with AMPSO buffer at pH 9.4 and different elution salts. Empty (red line), full (blue line) and mix of full and empty (violet line) rAAV8 particles analyzed in AEX mode by using three different elution salts with an elution method according to Example 2; E = empty rAAV8 particle, F = full rAAV8 particle, Rs = peak resolution, F/E = full/empty ratio.

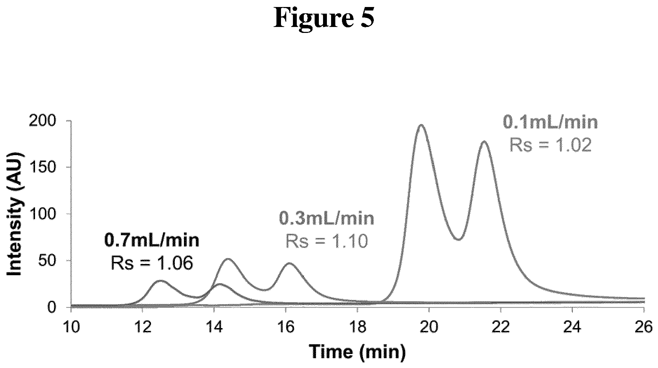

**Figure 5****.** Results with AMPSO buffer at pH 9.4 and TMAC as elution salt at different mobile phase flow rates. Mixture of full and empty rAAV8 particles analyzed in AEX mode by using three different flow rates, namely 0.7 mL/min (grey line), 0.3 mL/min (green line), and 0.1 mL/min (orange line) with an elution method according to Example 2.

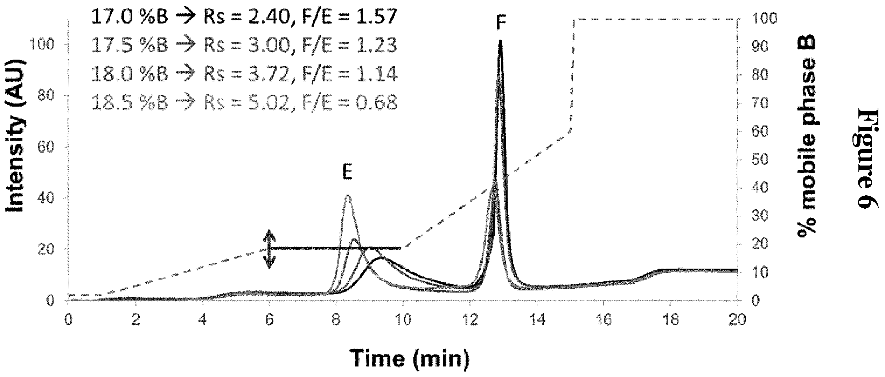

**Figure 6****.** Overlay of the chromatograms with different isocratic conditions. Mix of full and empty rAAV8 particles analyzed in AEX mode by using the AMPSO buffer pH 9.4 in combination with TMAC as eluent salt and the elution mode according to the invention with a 4-min isocratic step (red line) hold at different % (v/v) B, namely 17 % (v/v) B (black line), 17.5 % (v/v) B (blue line), 18 % (v/v) B (pink line), and 18.5 % (v/v) B (green line); E = empty rAAV8 particle, F = full rAAV8 particle, Rs = peak resolution, F/E = full/empty ratio.

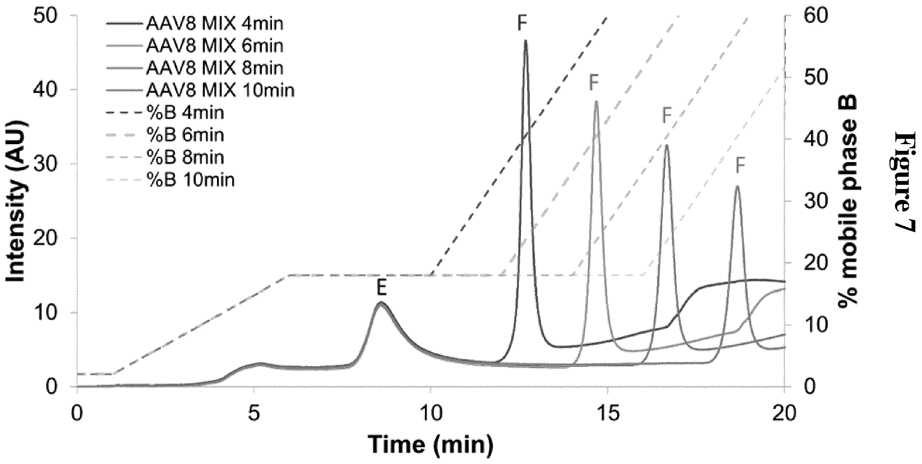

**Figure 7****.** Overlay of the chromatograms with different duration of the second isocratic step. Mixture of full and empty rAAV8 particles analyzed in AEX mode by using the AMPSO buffer pH 9.4 in combination with TMAC as eluent salt and the elution mode according to the invention including an isocratic step hold at 18 % (v/v) B for 4 (blue line), 6 (grey line), 8 (green line) and 10 (orange line) minutes; E = empty rAAV8 particle, F = full rAAV8 particle.

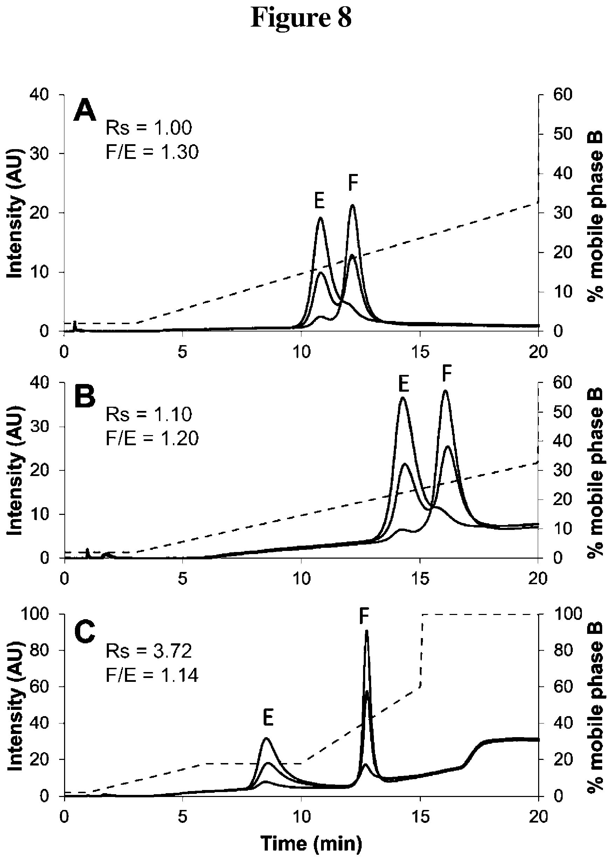

**Figure 8****.** Empty (red line), full (blue line) and mixture of full and empty (violet line) rAAV8 particles analyzed in AEX mode by using three different elution strategies, according to Example 2 (comparative) (A), according to Example 3 (comparative) (B), and according to Example 4 (according to the current invention); E = empty rAAV8 particle, F = full rAAV8 particle, Rs = peak resolution, F/E = full/empty ratio.

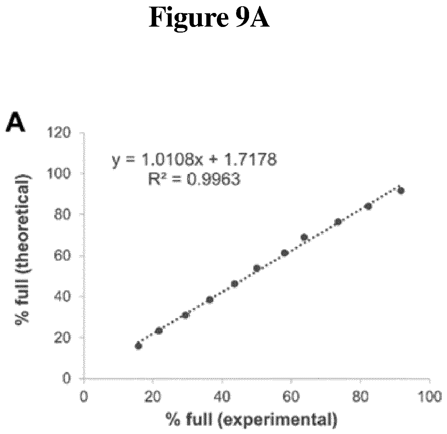

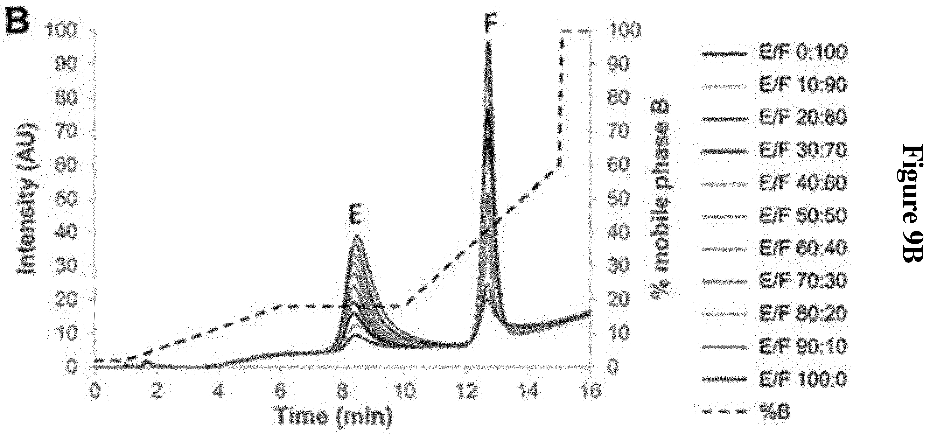

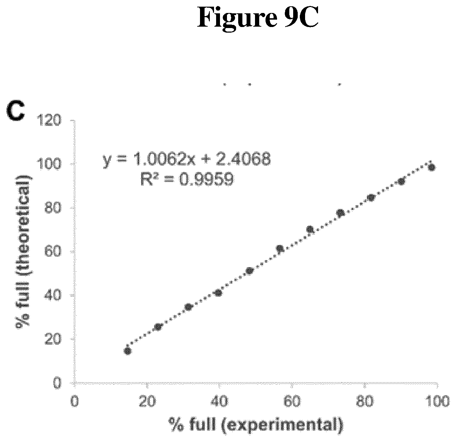

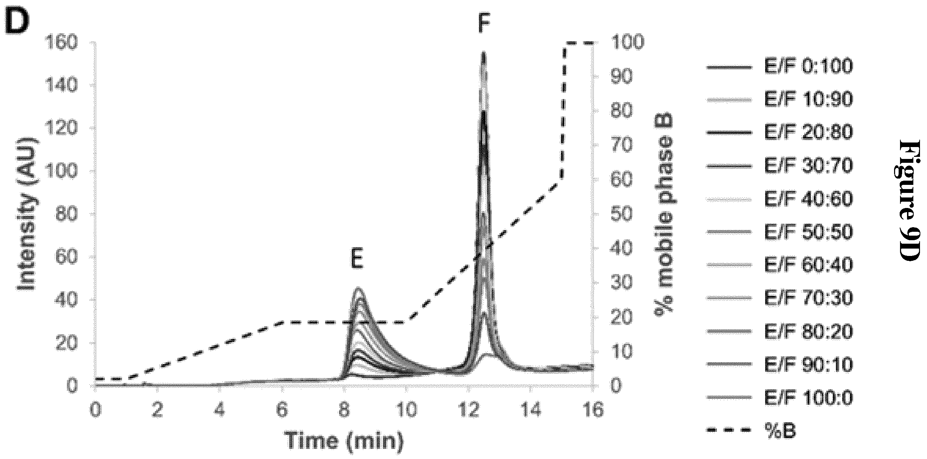

**Figure 9****.** Quantification of % full particles in different empty and full rAAV8 particle mixtures obtained from two different providers, namely Virovek (A-B) and Sirion (C-D). An elution method according to the current invention was applied with a second isocratic step at 18.5 % (v/v) B for the Sirion samples and 18 % (v/v) B for the Virovek samples; E = empty rAAV8 particle, F = full rAAV8 particle, F/E = full/empty ratio.

### Detailed Description of Embodiments of the Invention

### DEFINITIONS

It must be noted that as used herein and in the appended claims, the singular forms "a", "an", and "the" include plural reference unless the context clearly dictates otherwise. Thus, for example, reference to "a cell" includes a plurality of such cells and equivalents thereof known to those skilled in the art, and so forth. As well, the terms "a" (or "an"), "one or more" and "at least one" can be used interchangeably herein. It is also to be noted that the terms "comprising", "including", and "having" can be used interchangeably.

The term "about" denotes a range of +/- 20 % of the thereafter following numerical value. In certain embodiments, the term about denotes a range of +/- 10 % of the thereafter following numerical value. In certain embodiments, the term about denotes a range of +/- 5 % of the thereafter following numerical value.

The terms "comprise(s)," "include(s)," "having," "has," "can," "contain(s)" and variants thereof, as used herein, are intended to be open-ended transitional phrases, terms or words that do not preclude the possibility of additional acts or structures. The term "comprising" also encompasses the term "consisting of". The present disclosure also contemplates other embodiments "comprising," "consisting of" and "consisting essentially of," the embodiments or elements presented herein, whether explicitly set forth or not.

The terms "empty particle" and "empty recombinant AAV particle", which can be used interchangeably, denote an AAV particle that has an AAV protein shell but that lacks in whole or in part a nucleic acid that encodes a protein or is transcribed into a transcript of interest flanked by AAV ITRs, i.e. a vector. Accordingly, the empty particle does not function to transfer a nucleic acid that encodes a protein or is transcribed into a transcript of interest into a target cell.

The term "endogenous" denotes that something is naturally occurring within a cell; naturally produced by a cell; likewise, an "endogenous gene locus/cell-endogenous gene locus" is a naturally occurring locus in a cell.

As used herein, the term "exogenous" indicates that a nucleotide sequence does not originate from a specific cell and is introduced into said cell by DNA delivery methods, e.g., by transfection, electroporation, or transduction by viral vectors. Thus, an exogenous nucleotide sequence is an artificial sequence wherein the artificiality can originate, e.g., from the combination of subsequences of different origin (e.g. a combination of a recombinase recognition sequence with an SV40 promoter and a coding sequence of green fluorescent protein is an artificial nucleic acid) or from the deletion of parts of a sequence (e.g. a sequence coding only the extracellular domain of a membrane-bound receptor or a cDNA) or the mutation of nucleobases. The term "endogenous" refers to a nucleotide sequence originating from a cell. An "exogenous" nucleotide sequence can have an "endogenous" counterpart that is identical in base compositions, but where the sequence is becoming an "exogenous" sequence by its introduction into the cell, e.g., via recombinant DNA technology.

The terms "full particle" and "full recombinant AAV particle", which can be used interchangeably, denote an AAV particle that has an AAV protein shell and therein encapsidated a nucleic acid that encodes a protein or is transcribed into a transcript of interest flanked by AAV ITRs, i.e. a vector. Accordingly, the full particle can transfer the encapsidated nucleic acid that encodes a protein or is transcribed into a transcript of interest into a target cell.

The terms "full to empty ratio" and "full recombinant AAV particle to empty recombinant AAV particle ratio", which can be used interchangeably, denotes the mathematical ratio of the number of full recombinant AAV particles to the total number of recombinant AAV particles (full and empty) in a recombinant AAV particle containing sample or in a recombinant AAV particle preparation. As the number of full recombinant AAV particles can be at most the same as the total number of recombinant AAV particles the ratio can be at most 1. Generally, the ratio is less than 1 and is expressed as percentage. The number of full recombinant AAV particles is determined by determining the number of recombinant AAV particle encapsidated nucleic acid in the sample or preparation. This can be done by PCR, especially digital droplet PCR (ddPCR). The total number of recombinant AAV particles is determined by determining the number capsid proteins in the sample or preparation. This can be done by ELISA, especially by a capsid protein specific ELISA.

A "recombinant AAV vector" is derived from the wild-type genome of a virus, such as AAV by using molecular biological methods to remove the wild type genome from the virus (e.g., AAV), and replacing it with a non-native nucleic acid, such as a nucleic acid transcribed into a transcript or that encodes a protein. Typically, for AAV one or both inverted terminal repeat (ITR) sequences of the wild-type AAV genome are retained in the recombinant AAV vector. A "recombinant" AAV vector is distinguished from a wild-type viral AAV genome, since all or a part of the viral genome has been replaced with a non-native (i.e., heterologous) sequence with respect to the viral genomic nucleic acid. Incorporation of a non-native sequence therefore defines the viral vector (e.g., AAV) as a "recombinant" vector, which in the case of AAV can be referred to as a "rAAV vector."

A recombinant vector (e.g., AAV) sequence can be packaged - referred to herein as a "particle" - for subsequent infection (transduction) of a cell, ex vivo, in vitro or in vivo. Where a recombinant vector sequence is encapsulated or packaged into an AAV particle, the particle can also be referred to as a "rAAV". Such particles include proteins that encapsulate or package the vector genome. Particular examples include viral envelope proteins, and in the case of AAV, capsid proteins, such as AAV VP1, VP2 and VP3.

As used herein, the term "serotype" is a distinction based on AAV capsids being serologically distinct. Serologic distinctiveness is determined based on the lack of cross-reactivity between antibodies to one AAV as compared to another AAV. Such cross-reactivity differences are usually due to differences in capsid protein sequences/antigenic determinants (e.g., due to VP1, VP2, and/or VP3 sequence differences of AAV serotypes). Despite the possibility that AAV variants including capsid variants may not be serologically distinct from a reference AAV or other AAV serotype, they differ by at least one nucleotide or amino acid residue compared to the reference or other AAV serotype.

Under the traditional definition, a serotype means that the virus of interest has been tested against serum specific for all existing and characterized serotypes for neutralizing activity and no antibodies have been found that neutralize the virus of interest. As more naturally occurring virus isolates are discovered and/or capsid mutants generated, there may or may not be serological differences with any of the currently existing serotypes. Thus, in cases where the new virus (e.g., AAV) has no serological difference, this new virus (e.g., AAV) would be a subgroup or variant of the corresponding serotype. In many cases, serology testing for neutralizing activity has yet to be performed on mutant viruses with capsid sequence modifications to determine if they are of another serotype according to the traditional definition of serotype. Accordingly, for the sake of convenience and to avoid repetition, the term "serotype" broadly refers to both serologically distinct viruses (e.g., AAV) as well as viruses (e.g., AAV) that are not serologically distinct that may be within a subgroup or a variant of a given serotype.

A "vector" refers to the portion of the recombinant plasmid sequence ultimately packaged or encapsulated, either directly or in form of a single strand or RNA, to form a viral (e.g., AAV) particle. In cases recombinant plasmids are used to construct or manufacture recombinant viral particles, the viral particle does not include the portion of the "plasmid" that does not correspond to the vector sequence of the recombinant plasmid. This non-vector portion of the recombinant plasmid is referred to as the "plasmid backbone", which is important for cloning and amplification of the plasmid, a process that is needed for propagation and recombinant virus production, but is not itself packaged or encapsulated into virus (e.g., AAV) particles. Thus, a "vector" refers to the nucleic acid that is packaged or encapsulated by a virus particle (e.g., AAV).

### RECOMBINANT CELL

Generally, for efficient as well as large-scale production of a recombinant AAV particle (rAAV particle) a cell expressing and, if possible, also secreting said rAAV particle. Such a cell is termed "recombinant cell" or "recombinant production cell".

For the generation of a "recombinant production cell" a suitable mammalian cell is transfected with the required nucleic acid sequences for producing said rAAV particle, including the required AAV helper functions.

For expression of a coding sequence, i.e. of an open reading frame, additional regulatory elements, such as a promoter and polyadenylation signal (sequence), are necessary. Thus, an open reading frame is operably linked to said additional regulatory elements for transcription. This can be achieved by integrating it into a so-called expression cassette. The minimal regulatory elements required for an expression cassette to be functional in a mammalian cell are a promoter functional in said mammalian cell, which is located upstream, i.e. 5', to the open reading frame, and a polyadenylation signal (sequence) functional in said mammalian cell, which is located downstream, i.e. 3', to the open reading frame. Additionally a terminator sequence may be present 3' to the polyadenylation signal (sequence). For expression, the promoter, the open reading frame/coding region and the polyadenylation signal sequence have to be arranged in an operably linked form.

Likewise, a nucleic acid that is transcribed into a non-protein coding RNA is called "RNA gene". Also for expression of an RNA gene, additional regulatory elements, such as a promoter and a transcription termination signal or polyadenylation signal (sequence), are necessary. The nature and localization of such elements depends on the RNA polymerase that is intended to drive the expression of the RNA gene. Thus, an RNA gene is normally also integrated into an expression cassette.

In case of an AAV particle, which is composed of different (monomeric) capsid polypeptides and a single stranded DNA molecule and which in addition requires other adenoviral helper functions for production and encapsulation, a multitude of expression cassettes differing in the contained open reading frames/coding sequences are required. In this case, at least an expression cassette for each of the transgene, the different polypeptides forming the capsid of the AAV vector, for the required helper functions as well as the VA RNA are required. Thus, individual expression cassettes for each of the helper functions E1A, E1B, E2A, E4orf6, the VA RNA, the rep and cap genes are required. HEK293 cells express the E1A and E1B helper functions constitutively.

### ADENO-ASSOCIATED VIRUS (AAV)

For a general review of AAVs and of the adenovirus or herpes helper functions see, Berns and Bohensky, Advances in Virus Research, Academic Press., 32 (1987) 243-306. The genome of AAV is described in Srivastava et al., J. Virol., 45 (1983) 555-564. In US 4,797,368 design considerations for constructing recombinant AAV vectors are described (see also WO 93/24641). Additional references describing AAV vectors are West et al., Virol. 160 (1987) 38-47; Kotin, Hum. Gene Ther. 5 (1994) 793-801; and Muzyczka J. Clin. Invest. 94 (1994) 1351. Construction of recombinant AAV vectors described in US 5,173,414; Lebkowski et al., Mol. Cell. Biol. 8 (1988) 3988-3996; Tratschin et al., Mol. Cell. Biol. 5 (1985) 3251-3260; Tratschin et al., Mol. Cell. Biol., 4 (1994) 2072-2081; Hermonat and Muzyczka Proc. Natl. Acad. Sci. USA 81 (1984) 6466-6470; Samulski et al. J. Virol. 63 (1989) 3822-3828.

An adeno-associated virus (AAV) is a replication-deficient parvovirus. It can replicate only in cells, in which certain viral functions are provided by a co-infecting helper virus, such as adenoviruses, herpesviruses and, in some cases, poxviruses such as vaccinia. Nevertheless, an AAV can replicate in virtually any cell line of human, simian or rodent origin provided that the appropriate helper viral functions are present.

Without helper viral genes being present, an AAV establishes latency in its host cell. Its genome integrates into a specific site in chromosome 19 [(Chr) 19 (q13.4)], which is termed the adeno-associated virus integration site 1 (AAVS1). For specific serotypes, such as AAV-2 other integration sites have been found, such as, e.g., on chromosome 5 [(Chr) 5 (p13.3)], termed AAVS2, and on chromosome 3 [(Chr) 3 (p24.3)], termed AAVS3.

AAVs are categorized into different serotypes. These have been allocated based on parameters, such as hemagglutination, tumorigenicity and DNA sequence homology. Up to now, more than 10 different serotypes and more than a hundred sequences corresponding to different clades of AAV have been identified.

The capsid protein type and symmetry determines the tissue tropism of the respective AAV. For example, AAV-2, AAV-4 and AAV-5 are specific to retina, AAV-2, AAV-5, AAV-8, AAV-9 and AAVrh-10 are specific for brain, AAV-1, AAV-2, AAV-6, AAV-8 and AAV-9 are specific for cardiac tissue, AAV-1, AAV-2, AAV-5, AAV-6, AAV-7, AAV-8, AAV-9 and AAV-10 are specific for liver, AAV-1, AAV-2, AAV-5 and AAV-9 are specific for lung.

Pseudotyping denotes a process comprising the cross packaging of the AAV genome between various serotypes, i.e. the genome is packaged with differently originating capsid proteins.

The wild-type AAV genome has a size of about 4.7 kb. The AAV genome further comprises two overlapping genes named rep and cap, which comprise multiple open reading frames (see, e.g., Srivastava et al., J. Viral., 45 (1983) 555-564; Hermonat et al., J. Viral. 51 (1984) 329-339; Tratschin et al., J. Virol., 51 (1984) 611-619). The Rep protein encoding open reading frame provides for four proteins of different size, which are termed Rep78, Rep68, Rep52 and Rep40. These are involved in replication, rescue and integration of the AAV. The Cap protein encoding open reading frame provides four proteins, which are termed VP1, VP2, VP3, and AAP. VP1, VP2 and VP3 are part of the proteinaceous capsid of the AAV particles. The combined rep and cap open reading frames are flanked at their 5'- and 3'-ends by so-called inverted terminal repeats (ITRs). For replication, an AAV requires in addition to the Rep and Cap proteins the products of the genes E1A, E1B, E4orf6, E2A and VA of an adenovirus or corresponding factors of another helper virus.

In the case of an AAV of the serotype 2 (AAV-2), for example, the ITRs each have a length of 145 nucleotides and flank a coding sequence region of about 4470 nucleotides. Of the ITR's 145 nucleotides 125 nucleotides have a palindromic sequence and can form a T-shaped hairpin structure. This structure has the function of a primer during viral replication. The remaining 20, non-paired, nucleotides are denoted as D-sequence.

The AAV genome harbors three transcription promoters P5, P19, and P40 (Laughlin et al., Proc. Natl. Acad. Sci. USA 76 (1979) 5567-5571) for the expression of the rep and cap genes.

The ITR sequences have to be present in cis to the coding region. The ITRs provide a functional origin of replication (ori), signals required for integration into the target cell's genome, and efficient excision and rescue from host cell chromosomes or recombinant plasmids. The ITRs further comprise origin of replication like-elements, such as a Rep-protein binding site (RBS) and a terminal resolution site (TRS). It has been found that the ITRs themselves can have the function of a transcription promoter in an AAV vector (Flotte et al., J. Biol. Chem. 268 (1993) 3781-3790; Flotte et al., Proc. Natl. Acad. Sci. USA 93 (1993) 10163-10167).

For replication and encapsidation, respectively, of the viral single-stranded DNA genome an in trans organization of the rep and cap gene products are required.

The rep gene locus comprises two internal promoters, termed P5 and P19. It comprises open reading frames for four proteins. Promoter P5 is operably linked to a nucleic acid sequence providing for non-spliced 4.2 kb mRNA encoding the Rep protein Rep78 (chromatin nickase to arrest cell cycle), and a spliced 3.9 kb mRNA encoding the Rep protein Rep68 (site-specific endonuclease). Promoter P19 is operably linked to a nucleic acid sequence providing for a non-spliced mRNA encoding the Rep protein Rep52 and a spliced 3.3 kb mRNA encoding the Rep protein Rep40 (DNA helicases for accumulation and packaging).

The two larger Rep proteins, Rep78 and Rep68, are essential for AAV duplex DNA replication, whereas the smaller Rep proteins, Rep52 and Rep40, seem to be essential for progeny, single-strand DNA accumulation (Chejanovsky & Carter, Virology 173 (1989) 120-128).

The larger Rep proteins, Rep68 and Rep78, can specifically bind to the hairpin conformation of the AAV ITR. They exhibit defined enzyme activities, which are required for resolving replication at the AAV termini. Expression of Rep78 or Rep68 could be sufficient for infectious particle formation (Holscher, C., et al. J. Virol. 68 (1994) 7169-7177 and 69 (1995) 6880-6885).

It is deemed that all Rep proteins, primarily Rep78 and Rep68, exhibit regulatory activities, such as induction and suppression of AAV genes as well as inhibitory effects on cell growth (Tratschin et al., Mol. Cell. Biol. 6 (1986) 2884-2894; Labow et al., Mol. Cell. Biol., 7 (1987) 1320-1325; Khleif et al., Virology, 181 (1991) 738-741).

Recombinant overexpression of Rep78 results in phenotype with reduced cell growth due to the induction of DNA damage. Thereby the host cell is arrested in the S phase, whereby latent infection by the virus is facilitated (Berthet, C., et al., Proc. Natl. Acad. Sci. USA 102 (2005) 13634-13639).

Tratschin et al. reported that the P5 promoter is negatively auto-regulated by Rep78 or Rep68 (Tratschin et al., Mol. Cell. Biol. 6 (1986) 2884-2894). Due to the toxic effects of expression of the Rep protein, only very low expression has been reported for certain cell lines after stable integration of AAV (see, e.g., Mendelson et al., Virol. 166 (1988) 154-165).

The cap gene locus comprises one promoter, termed P40. Promoter P40 is operably linked to a nucleic acid sequence providing for 2.6 kb mRNA, which, by alternative splicing and use of alternative start codons, encodes the Cap proteins VP1 (87 kDa, non-spliced mRNA transcript), VP2 (72 kDa, from the spliced mRNA transcript), and VP3 (61 kDa, from alternative start codon). VP1 to VP3 constitute the building blocks of the viral capsid. The capsid has the function to bind to a cell surface receptor and allow for intracellular trafficking of the virus. VP3 accounts for about 90 % of total viral particle protein. Nevertheless, all three proteins are essential for effective capsid production.

It has been reported that inactivation of all three capsid proteins VP1 to VP3 prevents accumulation of single-strand progeny AAV DNA. Mutations in the VP1 amino-terminus ("Lip-negative" or "Inf-negative") still allows for assembly of single-stranded DNA into viral particles whereby the infectious titer is greatly reduced.

The AAP open reading frame is encoding the assembly activating protein (AAP). It has a size of about 22 kDa and transports the native VP proteins into the nucleolar region for capsid assembly. This open reading frame is located upstream of the VP3 protein encoding sequence.

In individual AAV particles, only one single-stranded DNA molecule is contained. This may be either the "plus" or "minus" strand. AAV viral particles containing a DNA molecule are infectious. Inside the infected cell, the parental infecting single strand is converted into a double strand, which is subsequently amplified. The amplification results in a large pool of double stranded DNA molecules from which single strands are displaced and packaged into capsids.

Adeno-associated viral (AAV) vectors can transduce dividing cells as well as resting cells. It can be assumed that a transgene introduced using an AAV vector into a target cell will be expressed for a long period. One drawback of using an AAV vector is the limitation of the size of the transgene that can be introduced into cells.

Viral vectors such as parvo-virus particles, including AAV serotypes and variants thereof, provide a means for delivery of nucleic acid into cells ex vivo, in vitro and in vivo, which encode proteins such that the cells express the encoded protein. AAVs are viruses useful as gene therapy vectors as they can penetrate cells and introduce nucleic acid/genetic material so that the nucleic acid/genetic material may be stably maintained in cells. In addition, these viruses can introduce nucleic acid/genetic material into specific sites, for example. Because AAV are not associated with pathogenic disease in humans, AAV vectors are able to deliver heterologous polynucleotide sequences (e.g., therapeutic proteins and agents) to human patients without causing substantial AAV pathogenesis or disease.

AAV particles used as vehicles for effective gene delivery possess a number of desirable features for such applications, including tropism for dividing and non-dividing cells. Early clinical experience with these vectors also demonstrated no sustained toxicity and immune responses were minimal or undetectable. AAV are known to infect a wide variety of cell types in vivo and in vitro by receptor-mediated endocytosis or by transcytosis. These vector systems have been tested in humans targeting retinal epithelium, liver, skeletal muscle, airways, brain, joints and hematopoietic stem cells.

Recombinant AAV particles do not typically include viral genes associated with pathogenesis. Such vectors typically have one or more of the wild-type AAV genes deleted in whole or in part, for example, rep and/or cap genes, but retain at least one functional flanking ITR sequence, as necessary for the rescue, replication, and packaging of the recombinant vector into an AAV particle. For example, only the essential parts of the vector e.g., the ITR and LTR elements, respectively, are included. An AAV vector genome would therefore include sequences required in cis for replication and packaging (e.g., functional ITR sequences).

Recombinant AAV vectors, as well as methods and uses thereof, include any viral strain or serotype. As a non-limiting example, a recombinant AAV vector can be based upon any AAV genome, such as AAV-1, -2, -3, -4, -5, -6, -7, -8, -9, -10, -11, -12, 2i8, AAV rh74 or AAV 7m8 for example. Such vectors can be based on the same strain or serotype (or subgroup or variant), or be different from each other. As a non-limiting example, a recombinant AAV vector based upon one serotype genome can be identical to one or more of the capsid proteins that package the vector. In addition, a recombinant AAV vector genome can be based upon an AAV (e.g., AAV2) serotype genome distinct from one or more of the AAV capsid proteins that package the vector. For example, the AAV vector genome can be based upon AAV2, whereas at least one of the three capsid proteins could be an AAV1, AAV3, AAV4, AAV5, AAV6, AAV7, AAV8, AAV9, AAV10, AAV11, AAV12, AAV-2i8, AAV rh74, AAV 7m8 or a variant thereof, for example. AAV variants include variants and chimeras of AAV1, AAV2, AAV3, AAV4, AAV5, AAV6, AAV7, AAV8, AAV9, AAV10, AAV11, AAV12, AAV-2i8, AAV rh74 and AAV 7m8 capsids.

In certain embodiments of all aspects and embodiments of the invention, the rAAV particle is derived from an AAV selected from the group consisting of AAV1, AAV2, AAV3, AAV4, AAV5, AAV6, AAV7, AAV8, AAV9, AAV10, AAV11, AAV12, AAV-2i8, AAV rh74 and AAV 7m8, as well as variants (e.g., capsid variants, such as amino acid insertions, additions, substitutions and deletions) thereof, for example, as set forth in WO 2013/158879, WO 2015/013313 and US 2013/0059732 (disclosing LK01, LK02, LK03, etc.).

In certain embodiments of all aspects and embodiments of the invention, the rAAV particle comprises a capsid sequence having 70 % or more sequence identity to an AAV1, AAV2, AAV3, AAV4, AAV5, AAV6, AAV7, AAV8, AAV9, AAV10, Rh10, Rh74, or 7m8 capsid sequence.

In certain embodiments of all aspects and embodiments of the invention, the rAAV particle comprises an ITR sequence having 70 % or more sequence identity to an AAV1, AAV2, AAV3, AAV4, AAV5, AAV6, AAV7, AAV8, AAV9, or AAV10 ITR sequence

Recombinant particles (e.g., rAAV particles) can be incorporated into pharmaceutical compositions. Such pharmaceutical compositions are useful for, among other things, administration and delivery to a subject in vivo or ex vivo. In certain embodiments, the pharmaceutical composition contains a pharmaceutically acceptable carrier or excipient. Such excipients include any pharmaceutical agent that does not itself induce an immune response harmful to the individual receiving the composition, and which may be administered without undue toxicity.

Protocols for the generation of adenoviral vectors have been described in US 5,998,205; US 6,228,646; US 6,093,699; US 6,100,242; WO 94/17810 and WO 94/23744.

### RECOMBINANT AAV PARTICLES (rAAV particles)

Different methods that are known in the art for generating rAAV particles. For example, transfection using AAV plasmid and AAV helper sequences in conjunction with co-infection with one AAV helper virus (e.g., adenovirus, herpesvirus, or vaccinia virus) or transfection with a recombinant AAV plasmid, an AAV helper plasmid, and an helper function plasmid. Non-limiting methods for generating rAAV particles are described, for example, in US 6,001,650, US 6,004,797, WO 2017/096039, and WO 2018/226887. Following recombinant rAAV particle production (i.e. particle generation in cell culture systems), rAAV particles can be obtained from the host cells and cell culture supernatant and purified.

For the generation of recombinant AAV particles, expression of the Rep and Cap proteins, the helper proteins E1A, E1B, E2A and E4orf6 as well as the adenoviral VA RNA in a single mammalian cell is required. The helper proteins E1A, E1B, E2A and E4orf6 can be expressed using any promoter as shown by Matsushita et al. (Gene Ther. 5 (1998) 938-945), especially the CMV IE promoter. Thus, any promoter can be used.

Generally, to produce recombinant AAV particles, different, complementing plasmids are co-transfected into a host cell. One of the plasmids comprises the transgene sandwiched between the two cis acting AAV ITRs. The missing AAV elements required for replication and subsequent packaging of progeny recombinant genomes, i.e. the open reading frames for the Rep and Cap proteins, are contained in trans on a second plasmid. The overexpression of the Rep proteins results in inhibitory effects on cell growth (Li, J., et al., J. Virol. 71 (1997) 5236-5243). Additionally, a third plasmid comprising the genes of a helper virus, i.e. E1, E4orf6, E2A and VA from adenovirus, is required for AAV replication.

To reduce the number of required plasmids, Rep, Cap and the adenovirus helper genes may be combined on a single plasmid.

Alternatively, the host cell may already stably express the E1 gene products. Such a cell is a HEK293 cell. The human embryonic kidney clone denoted as 293 was generated back in 1977 by integrating adenoviral DNA into human embryonic kidney cells (HEK cells) (Graham, F.L., et al., J. Gen. Virol. 36 (1977) 59-74). The HEK293 cell line comprises base pair 1 to 4344 of the adenovirus serotype 5 genome. This encompasses the E1A and E1B genes as well as the adenoviral packaging signals (Louis, N., et al., Virology 233 (1997) 423-429).

When using HEK293 cells the missing E2A, E4orf6 and VA genes can be introduced either by co-infection with an adenovirus or by co-transfection with an E2A-, E4orf6- and VA-expressing plasmid (see, e.g., Samulski, R.J., et al., J. Virol. 63 (1989) 3822-3828; Allen, J.M., et al., J. Virol. 71 (1997) 6816-6822; Tamayose, K., et al., Hum. Gene Ther. 7 (1996) 507-513; Flotte, T.R., et al., Gene Ther. 2 (1995) 29-37; Conway, J.E., et al., J. Virol. 71 (1997) 8780-8789; Chiorini, J.A., et al., Hum. Gene Ther. 6 (1995) 1531-1541; Ferrari, F.K., et al., J. Virol. 70 (1996) 3227-3234; Salvetti, A., et al., Hum. Gene Ther. 9 (1998) 695-706; Xiao, X., et al., J. Virol. 72 (1998) 2224-2232; Grimm, D., et al., Hum. Gene Ther. 9 (1998) 2745-2760; Zhang, X., et al., Hum. Gene Ther. 10 (1999) 2527-2537). Alternatively, adenovirus/AAV or herpes simplex virus/AAV hybrid vectors can be used (see, e.g., Conway, J.E., et al., J. Virol. 71 (1997) 8780-8789; Johnston, K.M., et al., Hum. Gene Ther. 8 (1997) 359-370; Thrasher, A.J., et al., Gene Ther. 2 (1995) 481-485; Fisher, J.K., et al., Hum. Gene Ther. 7 (1996) 2079-2087; Johnston, K.M., et al., Hum. Gene Ther. 8 (1997) 359-370).

In order to limit the transgene activity to specific tissues, i.e. to limit the site of integration the transgene can be operably linked to an inducible or tissue specific promoter (see, e.g., Yang, Y., et al. Hum. Gene. Ther. 6 (1995) 1203-1213).

### E1A, E1B, E2 and E4

The coding sequences of E1A and E1B (open reading frames) can be derived from a human adenovirus, such as, e.g., in particular of human adenovirus serotype 2 or serotype 5. An exemplary sequence of human Ad5 (adenovirus serotype 5) is found in GenBank entries X02996, AC_000008 and that of an exemplary human Ad2 in GenBank entry AC_000007. Nucleotides 505 to 3522 comprise the nucleic acid sequences encoding E1A and E1B of human adenovirus serotype 5. Plasmid pSTK146 as reported in EP 1 230 354, as well as plasmids pGS119 and pGS122 as reported in WO 2007/056994, can also be used a source for the E1A and E1B open reading frames.

E1A is the first viral helper gene that is expressed after adenoviral DNA enters the cell nucleus. The E1A gene encodes the 12S and 13S proteins, which are based on the same E1A mRNA by alternative splicing. Expression of the 12S and 13S proteins results in the activation of the other viral functions E1B, E2, E3 and E4. Additionally, expression of the 12S and 13S proteins force the cell into the S phase of the cell cycle. If only the E1A-derived proteins are expressed, the cell will die (apoptosis).

E1B is the second viral helper gene that is expressed. It is activated by the E1A-derived proteins 12S and 13S. The E1B gene derived mRNA can be spliced in two different ways resulting in a first 55 kDa transcript and a second 19 kDa transcript. The E1B 55 kDa protein is involved in the modulation of the cell cycle, the prevention of the transport of cellular mRNA in the late phase of the infection, and the prevention of E1A-induced apoptosis. The E1B 19 kDa protein is involved in the prevention of E1A-induced apoptosis of cells.

The E2 gene encodes different proteins. The E2A transcript codes for the single strand-binding protein (SSBP), which is essential for AAV replication

Also, the E4 gene encodes several proteins. The E4 gene derived 34 kDa protein (E4orf6) prevents the accumulation of cellular mRNAs in the cytoplasm together with the E1B 55 kDa protein, but also promotes the transport of viral RNAs from the cell nucleus into the cytoplasm.

### Adenoviral VA RNA

The viral associated RNA (VA RNA) is a non-coding RNA of adenovirus (Ad), regulating translation. The adenoviral genome comprises two independent copies: VAI (VA RNAI) and VAII (VA RNAII). Both are transcribed by RNA polymerase III (see, e.g., Machitani, M., et al., J. Contr. Rel. 154 (2011) 285-289) from a type 2 polymerases III promoter. For recombinant production, the adenoviral VA RNA gene can be driven by any promoter.

The structure, function, and evolution of adenovirus-associated RNA using a phylogenetic approach was investigated by Ma, Y. and Mathews, M.B. (J. Virol. 70 (1996) 5083-5099). They provided alignments as well as consensus VA RNA sequences based on 47 known human adenovirus serotypes.

VA RNAs, VAI and VAII, are consisting of 157-160 nucleotides (nt).

Depending on the serotype, adenoviruses contain one or two VA RNA genes. VA RNAI is believed to play the dominant pro-viral role, while VA RNAII can partially compensate for the absence of VA RNAI (Vachon, V.K. and Conn, G.L., Virus Res. 212 (2016) 39-52).

The VA RNAs are not essential, but play an important role in efficient viral growth by overcoming cellular antiviral machinery. That is, although VA RNAs are not essential for viral growth, VA RNA-deleted adenovirus cannot grow during the initial step of vector generation, where only a few copies of the viral genome are present per cell, possibly because viral genes other than VA RNAs that block the cellular antiviral machinery may not be sufficiently expressed (see Maekawa, A., et al. Nature Sci. Rep. 3 (2013) 1136).

Maekawa, A., et al. (Nature Sci. Rep. 3 (2013) 1136) reported efficient production of adenovirus vector lacking genes of virus-associated RNAs that disturb cellular RNAi machinery, wherein HEK293 cells that constitutively and highly express flippase recombinase were infected to obtain VA RNA-deleted adenovirus by FLP recombinase-mediated excision of the VA RNA locus.

The human adenovirus 2 VA RNAI corresponds to nucleotides 10586-10810 of GenBank entry AC_000007 sequence. The human adenovirus 5 VA RNAI corresponds to nucleotides 10579-10820 of GenBank entry AC_000008 sequence.

### METHODS FOR PRODUCING rAAV PARTICLES

Carter et al. have shown that the entire rep and cap open reading frames in the wild-type AAV genome can be deleted and replaced with a transgene (Carter, B. J., in "Handbook of Parvoviruses", ed. by P. Tijssen, CRC Press, pp. 155-168 (1990)). Further, it has been reported that the ITRs have to be maintained to retain the function of replication, rescue, packaging, and integration of the transgene into the genome of the target cell.

When cells comprising the respective viral helper genes are transduced by an AAV vector, or, vice versa, when cells comprising an integrated AAV provirus are transduced by a suitable helper virus, then the AAV provirus is activated and enters a lytic infection cycle again (Clark, K.R., et al., Hum. Gene Ther. 6 (1995) 1329-1341; Samulski, R.J., Curr. Opin. Genet. Dev. 3 (1993) 74-80).

Producer cells contain the rep and cap gene sequences, as well as the transgene cassette flanked by ITR sequences on one or more plasmids that are retained via drug selection. Production of rAAV particles in these cell lines generally occurs after their infection with the required helper functions. Therefore, cells are infected either with replication-competent AdV (usually wild type Ad5) or a plasmid comprising the respective helper genes to supply helper virus proteins and initiate rAAV particle production. A packaging cell line differs from a producer cell line as it only contains the rep and cap genes.

More generally, cells transfected or transduced with DNA for the recombinant production of AAV particles can be referred to as a "recombinant cell". Such a cell can be any mammalian cell that has been used as recipient of a nucleic acid (plasmid) encoding packaging proteins, such as AAV packaging proteins, a nucleic acid (plasmid) encoding helper proteins, and a nucleic acid (plasmid) that encodes a protein or is transcribed into a transcript of interest, i.e. a transgene placed between two AAV ITRs. The term includes the progeny of the original cell, which has been transduced or transfected. It is understood that the progeny of a single parental cell may not necessarily be completely identical in morphology or in genomic or total nucleic acid complement as the original parent, due to natural, accidental, or deliberate mutation.

Numerous cell growth media appropriate for sustaining cell viability or providing cell growth and/or proliferation are commercially available. Examples of such medium include serum free eukaryotic growth mediums, such as medium for sustaining viability or providing for the growth of mammalian (e.g., human) cells. Non-limiting examples include Ham's F12 or F12K medium (Sigma-Aldrich), FreeStyle (FS) F17 medium (Thermo-Fisher Scientific), MEM, DMEM, RPMI-1640 (Thermo-Fisher Scientific) and mixtures thereof. Such media can be supplemented with vitamins and/or trace minerals and/or salts and/or amino acids, such as essential amino acids for mammalian (e.g., human) cells.

For producing rAAV particles, three plasmids are co-transfected into a mammalian cell. The transgene plasmid encodes the expression cassette, which is cloned between the AAV ITRs, whereas rep and cap genes are provided in trans by co-transfecting a second, packaging plasmid (rep/cap plasmid) to ensure AAV replication and packaging. The third plasmid, also referred to as helper plasmid, contains the minimal helper virus factors, commonly adenoviral E2A, EV and VA genes, but lacking the AAV ITRs.

Diverse methods for the DNA transfer into mammalian cells have been reported in the art. These are all useful in the methods according to the current invention. In certain embodiments of all aspects and embodiments, electroporation, nucleofection, or microinjection for nucleic acid transfer/transfection is used. In certain embodiments of all aspects and embodiments, an inorganic substance (such as, e.g., calcium phosphate/DNA co-precipitation), a cationic polymer (such as, e.g., polyethylenimine, DEAE-dextran), or a cationic lipid (lipofection) is used for nucleic acid transfer/transfection is used. Calcium phosphate and polyethylenimine are the most commonly used reagents for transfection for nucleic acid transfer in larger scales (see, e.g., Baldi et al., Biotechnol. Lett. 29 (2007) 677-684), whereof polyethylenimine is preferred.

The growth in serum-free suspension culture and improvement of efficiency and reproducibility of transfection conditions using PEI as a transfection reagent permits ready scale-up the AAV production using shake-flasks, wave, or stirred-tank bioreactors.

The composition may comprise further plasmids or/and cells. Such plasmids and cells may be in contact with free PEI.

In addition to PEI, valproic acid (VPA) can be used to improve transfection efficiency. VPA, a branched short-chain fatty acid and inhibits histone deacetylase activity. Due to this reason, it is commonly added to mammalian cell culture as an enhancer of recombinant protein production.

Encoded AAV packaging proteins include, in certain embodiments of all aspects and embodiments, AAV rep and/or AAV cap. Such AAV packaging proteins include, in certain embodiments of all aspects and embodiments, AAV rep and/or AAV cap proteins of any AAV serotype.

Encoded helper proteins include, in certain embodiments of all aspects and embodiments, adenovirus E1A and E1B, adenovirus E2 and/or E4, VA RNA, and/or non-AAV helper proteins.

The cultivation can be performed using the generally used conditions for the cultivation of eukaryotic cells of about 37 °C, 95 % humidity and 8 vol.-% CO2. The cultivation can be performed in serum containing or serum free medium, in adherent culture or in suspension culture. The suspension cultivation can be performed in any fermentation vessel, such as, e.g., in stirred tank reactors, wave reactors, rocking bioreactors, shaker vessels or spinner vessels or so called roller bottles. Transfection can be performed in high throughput format and screening, respectively, e.g. in a 96 or 384 well format.

Methods according to the current invention can include AAV particles of any serotype, or a variant thereof. In certain embodiments of all aspects and embodiments, a recombinant AAV particle comprises any of AAV serotypes 1-12, an AAV VP1, VP2 and/or VP3 capsid protein, or a modified or variant AAV VP1, VP2 and/or VP3 capsid protein, or wild-type AAV VP1, VP2 and/or VP3 capsid protein. In certain embodiments of all aspects and embodiments, an AAV particle comprises an AAV serotype or an AAV pseudotype, where the AAV pseudotype comprises an AAV capsid serotype different from an ITR serotype.

Expression control elements include constitutive or regulatable control elements, such as a tissue-specific expression control element or promoter.

ITRs can be any of AAV2 or AAV6 or AAV8 or AAV9 serotypes, or a combination thereof. AAV particles can include any VP1, VP2 and/or VP3 capsid protein having 75 % or more sequence identity to any of AAV1, AAV2, AAV3, AAV4, AAV5, AAV6, AAV10, AAV11, AAV-2i8, AAV rh74 or AAV 7m8 VP1, VP2 and/or VP3 capsid proteins, or comprises a modified or variant VP1, VP2 and/or VP3 capsid protein selected from any of: AAV1, AAV2, AAV3, AAV4, AAV5, AAV6, AAV10, AAV11, AAV-2i8, AAV rh74 and AAV 7m8 AAV serotypes.

Following production of recombinant viral (e.g., AAV) particles, if desired, the viral (e.g., rAAV) particles can be purified and/or isolated from host cells using a variety of conventional methods. Such methods include column chromatography, CsCl gradients, iodixanol gradient and the like.

For example, a plurality of column purification steps such as purification over an anion exchange column, an affinity column and/or a cation exchange column can be used. (See, e.g., WO 02/12455 and US 2003/0207439). Alternatively, or in addition, an iodixanol or CsCl gradient steps can be used (see, e.g., US 2012/0135515; and US 2013/0072548). Further, if the use of infectious virus is employed to express the packaging and/or helper proteins, residual virus can be inactivated, using various methods. For example, adenovirus can be inactivated by heating to temperatures of approximately 60 °C for, e.g., 20 minutes or more. This treatment effectively inactivates the helper virus since AAV is heat stable while the helper adenovirus is heat labile.

An objective in the rAAV vector production and purification systems is to implement strategies to minimize/control the generation of production related impurities such as proteins, nucleic acids, and vector-related impurities, including wild-type/pseudo wild-type AAV species (wtAAV) and AAV-encapsulated residual DNA impurities.

Considering that the rAAV particle represents only a minor fraction of the biomass, rAAV particles need to be purified to a level of purity, which can be used as a clinical human gene therapy product (see, e.g., Smith P.H., et al., Mo. Therapy 7 (2003) 8348; Chadeuf G., et al, Mo. Therapy 12 (2005) 744; report from the CHMP gene therapy expert group meeting, European Medicines Agency EMEA/CHMP 2005, 183989/2004).

As an initial step, typically the cultivated cells that produce the rAAV particles are harvested, optionally in combination with harvesting cell culture supernatant (medium) in which the cells (suspension or adherent) producing rAAV particles have been cultured. The harvested cells and optionally cell culture supernatant may be used as is, as appropriate, lysed or concentrated. Further, if infection is employed to express helper functions, residual helper virus can be inactivated. For example, adenovirus can be inactivated by heating to temperatures of approximately 60 °C for, e.g., 20 minutes or more, which inactivates only the helper virus since AAV is heat stable while the helper adenovirus is heat labile.

The cells in the harvested cultivation broth are lysed by the method according to the current invention to release the rAAV particles. Concurrently during cell lysis or subsequently after cell lysis, a nuclease, such as, e.g., benzonase, is added to degrade contaminating DNA. Typically, the resulting lysate is clarified to remove cell debris, e.g. by filtering or centrifuging, to render a clarified cell lysate. In a particular example, lysate is filtered with a micron diameter pore size filter (such as a 0.1-10.0 µm pore size filter, for example, a 0.45 µm and/or pore size 0.2 µm filter), to produce a clarified lysate.

The lysate (optionally clarified) contains AAV particles (comprising rAAV vectors as well as empty capsids) and production/process related impurities, such as soluble cellular components from the host cells that can include, inter alia, cellular proteins, lipids, and/or nucleic acids, and cell culture medium components. The optionally clarified lysate is then subjected to purification steps to purify AAV particles (comprising rAAV vectors) from impurities using chromatography. The clarified lysate may be diluted or concentrated with an appropriate buffer prior to the first chromatography step.

After cell lysis, optional clarifying, and optional dilution or concentration, a plurality of subsequent and sequential chromatography steps can be used to purify rAAV particles.

The first chromatography step is preferably an affinity chromatography step using an AAV affinity chromatography ligand.

If the first chromatography step is affinity chromatography the second chromatography step can be anion exchange chromatography. Thus, in certain embodiments of all aspects and embodiments, rAAV particle purification is via affinity chromatography, followed by purification via anion exchange chromatography or/and cation exchange chromatography or/and size exclusion chromatography, in any order or sequence or combination.

The removal of empty capsids from full ones, for example, during downstream processing is based on their different isoelectric points (pI) in anion exchange chromatography. The average calculated pI across all serotypes is 5.9 for full capsids and 6.3 for empty capsids (Venkatakrishnan, B., et al., J. Virol. 87 (2013) 4974-4984).

Cation exchange chromatography functions to separate the AAV particles from cellular and other components present in the clarified lysate and/or column eluate from an affinity or size exclusion chromatography. Examples of strong cation exchange resins capable of binding rAAV particles over a wide pH range include, without limitation, any sulfonic acid based resin as indicated by the presence of the sulfonate functional group, including aryl and alkyl substituted sulfonates, such as sulfopropyl or sulfoethyl resins. Representative matrices include but are not limited to POROS HS, POROS HS 50, POROS XS, POROS SP, and POROS S (strong cation exchangers available from Thermo Fisher Scientific, Inc., Waltham, MA, USA). Additional examples include Capto S, Capto S ImpAct, Capto S ImpRes (strong cation exchangers available from GE Healthcare, Marlborough, MA, USA), and commercial DOWEX^{®}, AMBERLITE^{®}, and AMBERLYST^{®} families of resins available from Aldrich Chemical Company (Milliwaukee, WI, USA). Weak cation exchange resins include, without limitation, any carboxylic acid based resin. Exemplary cation exchange resins include carboxymethyl (CM), phospho (based on the phosphate functional group), methyl sulfonate (S) and sulfopropyl (SP) resins.

Anion exchange chromatography functions to separate AAV particles from proteins, cellular and other components present in the clarified lysate and/or column eluate from an affinity or cation exchange or size exclusion chromatography. Anion exchange chromatography can also be used to reduce and thereby control the amount of empty capsids in the eluate. For example, the anion exchange column having rAAV particle bound thereto can be washed with a solution comprising NaCl at a modest concentration (e.g., about 100-125 mM, such as 110-115 mM) and a portion of the empty capsids can be eluted in the flow through without substantial elution of the rAAV particles. Subsequently, rAAV particles bound to the anion exchange column can be eluted using a solution comprising NaCl at a higher concentration (e.g., about 130-300 mM NaCl), thereby producing a column eluate with reduced or depleted amounts of empty capsids and proportionally increased amounts of rAAV particles comprising an rAAV vector.

Exemplary anion exchange resins include, without limitation, those based on polyamine resins and other resins. Examples of strong anion exchange resins include those based generally on the quaternized nitrogen atom including, without limitation, quaternary ammonium salt resins such as trialkylbenzyl ammonium resins. Suitable exchange chromatography materials include, without limitation, MACRO PREP Q (strong anion-exchanger available from BioRad, Hercules, CA, USA); UNOSPHERE Q (strong anion-exchanger available from BioRad, Hercules, CA, USA); POROS 50HQ (strong anion-exchanger available from Applied Biosystems, Foster City, CA, USA); POROS XQ (strong anion-exchanger available from Applied Biosystems, Foster City, CA, USA); POROS SOD (weak anion-exchanger available from Applied Biosystems, Foster City, CA, USA); POROS 50PI (weak anion-exchanger available from Applied Biosystems, Foster City, CA, USA); Capto Q, Capto XQ, Capto Q ImpRes, and SOURCE 30Q (strong anion-exchanger available from GE healthcare, Marlborough, MA, USA); DEAE SEPHAROSE (weak anion-exchanger available from Amersham Biosciences, Piscataway, NJ, USA); Q SEPHAROSE (strong anion-exchanger available from Amersham Biosciences, Piscataway, NJ, USA). Additional exemplary anion exchange resins include aminoethyl (AE), diethylaminoethyl (DEAE), diethylaminopropyl (DEPE) and quaternary amino ethyl (QAE).

A manufacturing process to purify recombinant AAV particles intended as a product to treat human disease should achieve the following objectives: 1) consistent particle purity, potency and safety; 2) manufacturing process scalability; and 3) acceptable cost of manufacturing.

Exemplary processes for recombinant AAV particle purification are reported in WO 2019/006390.

Methods to determine infectious titer of rAAV particles containing a transgene are known in the art (see, e.g., Zhen et al., Hum. Gene Ther. 15 (2004) 709). Methods for assaying for empty capsids and rAAV particles with packaged transgenes are known (see, e.g., Grimm et al., Gene Therapy 6 (1999) 1322-1330; Sommer et al., Malec. Ther. 7 (2003) 122-128).

To determine the presence or amount of degraded/denatured capsid, purified rAAV particle can be subjected to SDS-polyacrylamide gel electrophoresis, consisting of any gel capable of separating the three capsid proteins, for example, a gradient gel, then running the gel until sample is separated, and blotting the gel onto nylon or nitrocellulose membranes. Anti-AAV capsid antibodies are then used as primary antibodies that bind to denatured capsid proteins (see, e.g., Wobus et al., J. Viral. 74

(2000) 9281-9293). A secondary antibody that binds to the primary antibody contains a means for detecting the primary antibody. Binding between the primary and secondary antibodies is detected semi-quantitatively to determine the amount of capsids. Another method would be analytical HPLC with a SEC column or analytical ultracentrifuge.

### THE METHOD ACCORDING TO THE INVENTION

Twelve different AAV serotypes are known and characterized by specific target tissue tropism.

An AAV is composed of the protein capsid, which contains a viral genome. The capsid is comprised of around 60 copies of three viral proteins VP1, VP2, VP3 (at 1:1:10 ratio, respectively) assembled into an icosahedron of 26-nm diameter. The genome is composed of single-stranded DNA of about 4.7 kb. The structure of the genome is relatively simple as it is composed of three genes flanked by two inverted terminal repeats (ITRs) at the ends (Straus, S.E., et al., Proc. Natl. Acad. Sci. 73 (1976) 742-746). The first gene is the Rep gene, which encodes four proteins (Rep78, Rep68, Rep52 and Rep40), produced from the same sequence, but from different promoters and through alternative splicing (Dismuke, D., et al., Curr. Gene Ther. 13 (2014) 434-452). They are useful for targeting viral integration, viral replication, transcription, and packaging of viral DNA into the viral capsid (Dismuke, D., et al., Curr. Gene Ther. 13 (2014) 434-452). The second gene is the Cap gene, which encodes the three viral capsid proteins (VP1, VP2, and VP3) that are different despite the same genetic sequence, due to translation from different start codons and alternative splicing. The third gene encodes the assembly activation protein (AAP) and is located within the Cap coding sequence (Dismuke, D., et al., Curr. Gene Ther. 13 (2014) 434-452; Weitzman, M.D., et al., Proc. Natl. Acad. Sci. 91 (1994) 5808-5812).

Given its simplicity, the wild-type AAV genome can be easily engineered and replaced by an artificial AAV genome, resulting in an artificial recombinant AAV (rAAV) missing the viral DNA encoding for the viral proteins (Rep and Cap). Indeed, the ITR sequences of the viral genome are conserved to maintain the transcriptional activity, while the rest of the viral sequence is replaced by an expression cassette containing the therapeutic gene, which is called the transgene (Naso, M.F., et al., BioDrugs. 31 (2017) 317-334). The transgene allows the expression of a desired therapeutic entity, such as, e.g., a polypeptide or protein, that might be, for example, a missing or non-functional protein related to a specific pathological state (Maguire, A.M., et al., Mol. Ther. 29 (2021) 442-463).

During the production of rAAV particles, the artificial AAV genome may not be integrated properly in the AAV capsid. Thereby a mixture of empty rAAV particles and full rAAV particles is produced. The fraction of empty rAAV particles may correspond to 10 % up to 90 % of total rAAV particles (Flotte, T.R., Hum. Gene Ther. 28 (2017) 147-148). Empty rAAV particles and partially filled rAAV particles are therefore product-related impurities that may reduce the effective concentration of the final drug, compete for binding sites and decrease the efficacy of the drug (Gao, K., et al., Mol. Ther. - Methods Clin. Dev. 1 (2014) 9). According to the FDA, these impurities need to be monitored and reported as a full/empty (F/E) ratio (FDA Guidance for Industry: Chemistry, Manufacturing, and Control (CMC) Information for Human Gene Therapy Investigational New Drug Applications (INDs), (2020)).

Because of the similarity to each other, empty rAAV particles are difficult to avoid or eliminate (Schnoedt, M. and Buening, H., Hum. Gene Ther. Methods. 28 (2017) 101-108). Full and empty rAAV particles have the same size. However, as full rAAV particles are loaded with negatively charged DNA, they have a slightly lower pI than empty rAAV particles. Generally, a difference in the range of 0.4 pH units can be observed. This feature is the basis for the separation and quantification of full and empty rAAV particles in the method according to the current invention.

The invention is based, at least in part, on the finding that the combination of linear gradients with isocratic steps allowed the unprecedented separation of full/empty rAAV particles, in one preferred embodiment rAAV particles of the serotype 8 (rAAV8). The method according to the invention allows for a precise and accurate baseline separation and quantification of full and empty rAAV particles. One application of the method according to the invention is a high-throughput method or/ quality control (QC) method for rAAV.

The following is presented using a recombinant AAV particle with capsid of the serotype 8. This is presented solely to exemplify the invention and shall not be construed as limitation of the general applicability of the method according to the current invention. The true scope is set forth in the appended claims.

Four strong anion exchange adsorbents, all functionalized with a positively charged quaternary group and differing in terms of matrix composition (monolith or non-porous beads) were tested with respect to their efficacy in resolving empty and full rAAV8 particles.

The column material was stainless steel, PEEK, or PEEK-lined stainless steel (SS), which should not have an influence on the separation properties. PEEK denotes polyether ether ketone. PEEK tubing has become a standard item in the operation of many HPLC systems and exhibits high column strength, as well as high tensile strength and high flexural modulus, making it perfect for catheters and other tubing applications requiring good torque response and pushability. Extruded PEEK tubing is used in medical applications where exceptionally high rigidity is needed.

**Table 1. List of chromatographic columns and their properties (chemistry: quaternary positively charged N-based functional group for all columns). PEEK and SS stand for Polyether Ether Ketone and Stainless Steel, respectively.**

| **column** | **name** | **provider** | **stationary phase** | **dimensions [mm]** | **particle size [µm]** |
|---|---|---|---|---|---|
| PP | ProPac SAX-10 | Thermo | Nonporous PEEK | 50 x 4 | 10 |
| PS | ProSwift SAX-1S | Thermo | Monolith PEEK-lined SS | 50 x 4.6 | - |
| AX | Bio-SAX | Agilent | Nonporous PEEK | 50 x 4.6 | 5 |
| QS | TSKgel Q-STAT | Tosoh | Nonporous SS tubing, PEEK frits | 100 x 4.6 | 7 |

To work within the column pressure limits, the flow rate was adjusted to 0.7 mL/min used with PP and AX columns and 0.5 mL/min and 0.35 mL/min for the QS and the PS columns, respectively. The method of Example 2 was used.

As shown Figure 1, appropriate peak resolution (Rs) was obtained only with the PP (Rs = 1.07) and PS (Rs = 1.00) columns.

The QS column comprises a chromatography material based on a non-porous polymer. In more detail, the stationary phase/chromatography material is a monodisperse, non-porous methacrylate-based resin particle of which the surface consists of an open access network of multi-layered anion exchange groups. The surface has been modified with a quaternary ammonium. QS is a strong anion exchanger.

The AX column comprises a chromatography material based on a synthetic stationary phase consisting of a highly cross-linked particle core of a copolymer based on poly(styrene divinylbenzene) (PS-DVB) coated with a hydrophilic, polymeric layer and a thereon chemically bound densely packed quaternary ammonium functional groups. AX is a strong anion exchanger.

The AX column has been reported to be useful in the analysis of full/empty capsid ratios in Adeno-Associated Virus 1 and 6 serotypes using biocompatible liquid chromatography (Liau, B, Agilent Application Note DE10415602, 5994-4589EN, February 2022).

The PS column comprises a chromatography material based on polymeric monoliths prepared by an in situ polymerization process. The monolith is a single cylindrical polymer rod containing an uninterrupted, interconnected network of through pores, which are also called channels. The material can be a methacrylate polymer. The monoliths consist of aggregates of globules. Most of the globules are engineered to be less than 500 nm. The globules are essentially non-porous. The pore volume is about 60 % of the column volume. The range of modal pore size is from approximately 1.6 µm to 2.7 µm with about 2 µM being the most abundant pore size. The surface has been modified with a quaternary amine. PS is a strong anion exchanger.

In one preferred embodiment of all aspects and embodiments according to the current invention, the stationary phase/chromatography material is a synthetic stationary phase consisting of a polymeric monolith. In certain embodiments, the monolith is a methacrylate polymer. In certain embodiments, the monolith is functionalized with quaternary amine functional groups.

The PP column comprises a chromatography material based on a synthetic stationary phase consisting of a highly cross-linked particle core of a copolymer based on ethylvinylbenzene and divinylbenzene (EVB-DVB) coated with a cross-linked hydrophilic boundary layer and a thereon grafted linear anion exchange phase with quaternary ammonium functional groups. In more detail, the PP stationary phase consists of microporous ethylvinylbenzene cross-linked with 55 % divinylbenzene polymer substrate, which is covered with a highly hydrophilic, neutral polymer forming a hydrophilic layer onto which a controlled polymer chain bearing quaternary ammonium groups has been grafted to introduce the anion exchange functionality. PP is a strong anion exchanger.

In one preferred embodiment of all aspects and embodiments according to the current invention, the stationary phase/chromatography material is a synthetic stationary phase consisting of a highly cross-linked particle core of a copolymer based on styrene and divinylbenzene (EVB-DVB) coated with a cross-linked hydrophilic boundary layer and a thereon grafted linear anion exchange phase with quaternary ammonium functional groups. In certain embodiments, the stationary phase consists of microporous ethylvinylbenzene cross-linked with 55 % divinylbenzene polymer substrate, which is covered with a highly hydrophilic, neutral polymer forming a hydrophilic layer onto which a controlled polymer chain bearing quaternary ammonium groups has been grafted. In certain embodiments, the solid phase are particles. In certain embodiments, the particle size, i.e. the particle diameter, of the solid phase is about 10 µm.

In the following, the PP column is used to exemplify the method according to the current invention.

It has been found that rAAV8 particle dilutions performed in a buffer comprising PBS (phosphate buffered saline; 137 mM NaCl, 2.7 mM KCl, 10 mM Na₂HPO₄, 1.8 mM KH₂PO₄) supplemented with 0.001 % (w/v) poloxamer 188 resulted in more stable samples over time. Only limited variations of the rAAV8 full (RSD = 8.6%) and empty (RSD = 8.3%) peak areas were observed in such dilution conditions, and the measurement of the full/empty ratio was much more precise (RSD = 3.4%) compared to water as diluent.

**Table 2. Summary of the results of the sample stability study.**

| | **t_{R} empty (min)** | **t_{R} full (min)** | **F/E ratio** | **Area empty (AU)** | **Area full (AU)** | **Resolution** |
|---|---|---|---|---|---|---|
| **Dilution in water** | | | | | | |
| Dav 0 | 10.88 | 12.23 | 0.89 | 21.21 | 17.55 | 1.03 |
| Day 1 | 10.83 | 12.17 | 1.06 | 13.85 | 14.62 | 1.09 |
| Day 3 | 10.60 | 11.95 | 1.37 | 9.11 | 14.04 | 1.08 |
| Day 7 | 10.47 | 11.84 | 1.38 | 9.60 | 15.02 | 1.08 |
| SD | 0.19 | 0.18 | 0.24 | 5.60 | 1.55 | 0.03 |
| Average | 10.69 | 12.05 | 1.18 | 13.44 | 15.31 | 1.07 |
| RSD (%) | 1.8% | 1.5% | 20.5% | 41.7% | 10.1% | 2.5% |

| **Dilution in PBS buffer** | | | | | | |
|---|---|---|---|---|---|---|
| Day 0 | 10.51 | 11.87 | 1.09 | 28.40 | 34.24 | n.a. |
| Day 1 | 10.55 | 11.87 | 1.09 | 28.56 | 34.18 | n.a. |
| Day 3 | 10.47 | 11.78 | 1.12 | 27.01 | 33.03 | n.a. |
| Day 7 | 10.32 | 11.64 | 1.17 | 23.75 | 28.38 | n.a. |
| SD | 0.10 | 0.11 | 0.04 | 2.23 | 2.78 | / |
| Average | 10.46 | 11.80 | 1.12 | 26.93 | 32.46 | / |
| RSD (%) | 0.9% | 1.0% | 3.4% | 8.3% | 8.6% | / |

The poloxamer-supplemented buffer was therefore selected as preferential diluting solvent and the remaining rAAV8 analyses were always performed within 3 days from sample preparation to limit variability related to sample stability.

Different buffer salts, pH values, and salts were tested.

**Table 3. List of buffer salts and their chemical structures.**

| **buffer salt** | **abbreviation** | **MW [g/mol]** | **pKₐ [25 °C]** | **structure** |
|---|---|---|---|---|
| 2-Amino-2-methyl-1,3-propane diol | AMPD | 105.15 | 8.8 | 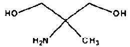 |
| 1,3-Bis[tris(hydroxymethyl) methylamino]propane | BTP | 282.33 | 9.0 | 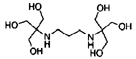 |
| N-(1,1-Dimethyl-2-hydroxyethyl)-3-amino-2-hydroxypropane sulfonic acid | AMPSO | 227.28 | 9.0 | 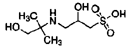 |
| 2-(Cyclohexylamino) ethane sulfonic acid | CHES | 207.29 | 9.5 | 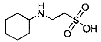 |
| 3-(Cyclohexylamino)-2-hydroxy-1-propane sulfonic acid | CAPSO | 237.32 | 9.6 | 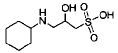 |

The reference BTP buffer was compared to four alternative biological buffers having different pKa values and chemical structures (see Table 2), namely AMPD, AMPSO, CHES, and CAPSO. The chromatograms obtained with these different buffered mobile phases using an elution method according to Example 2 are shown in Figure 2. The full/empty ratio (F/E) and the chromatographic resolution (Rs) were determined. It can be seen that the full/empty ratios were not significantly affected (ranging from 1.08 to 1.28) using the different buffers. However, only for the BTP buffer and the AMPSO buffer a resolution could be obtained and an Rs value calculated (Rs = 0.80 and 0.84, respectively).

In certain embodiments, the buffer in the method according to the invention is 1,3-bis[tris(hydroxymethyl)methylamino]propane.

In one preferred embodiment of all aspects and embodiments of the invention, the buffer in the method according to the invention is N-(1,1-Dimethyl-2-hydroxyethyl)-3-amino-2-hydroxypropane sulfonic acid.

In the following, the AMPSO buffer is used to exemplify the method according to the current invention.

Mobile phase consisting of AMPSO buffer were evaluated at different pH values ranging from pH 8.6 to pH 9.4 in 0.2 pH units intervals. An elution method according to Example 2 was used. As shown in Figure 3, a slight decrease of the retention times was observed when increasing the mobile phase pH. This behavior is in line with the AMPSO pKa being equal to 9. When using mobile phases buffered at pH values lower than its pKa (here 8.6 and 8.8), a higher amount of the AMPSO neutral form is present in solution, while with mobile phases buffered at pH values above the pKa (here 9.2 and 9.4), a higher amount of the negatively-charged form of the AMPSO is expected in solution. As only the negatively charged form of the AMPSO buffer is competing with the analytes for the binding to the positively charged stationary phase, a slight decrease of the retention times was observed when increasing the mobile phase pH. Although the full/empty ratios were not significantly affected by a pH variation (ranging from 1.00 to 1.09), a higher resolution was obtained at pH 9.4 (Rs = 0.93) as compared to pH 9.0 (Rs = 0.70).

In certain embodiment of all aspects and embodiments of the method according to the current invention, the mobile phases have a pH value of 9.0 or higher. In certain embodiments, the mobile phases have a pH value of 9.0 to 10.0. In certain embodiments, the mobile phases have a pH value of 9.2 to 9.6. In one preferred embodiment, the mobile phases have a pH value of about 9.4.

In the following, a pH value of 9.4 is used to exemplify the method according to the current invention.

Three different salt types were tested as eluents for separating rAAV8 full and empty particles, NaCl, KCl and tetramethyl ammonium chloride (TMAC). In Figure 4, the corresponding chromatograms obtained by using the different elution salts are shown.

Notably, although the full/empty ratios were not significantly impacted (ranging from 0.96 to 1.09), the use of an organic chloride, TMAC, resulted in a better resolution (Rs = 1.14) in comparison to the inorganic salts NaCl (Rs = 0.93) and KCl (Rs = 0.90).

In certain embodiments of all aspects and embodiments of the method according to the invention, the salt causing the elution of the rAAV particle is an organic salt. In certain embodiments, the organic salt is an organic chloride. In certain embodiments, the organic salt is an organic chloride with a molecular weight below 200 g/mol. In certain embodiments, the organic salt is an organic chloride with a molecular weight below 170 g/mol. In certain embodiments, the organic salt is tetraethyl ammonium chloride. In one preferred embodiment, the organic salt is tetramethyl ammonium chloride.

In the following, tetramethyl ammonium chloride is used as elution salt to exemplify the method according to the current invention.

Three different mobile phase flow rates, namely 0.7 mL/min, 0.3 mL/min, and 0.1 mL/min were tested. The results are shown in Figure 5.

A flow rate of 0.3 mL/min was selected as the best compromise between chromatographic resolution, peak intensity, and mobile phase consumption.

Large proteins show a specific elution behavior under reversed phase liquid chromatography (RPLC) conditions, where the retention is extremely sensitive to mobile phase composition (see, e.g., Snyder, L.R., et al., Anal. Chem. 55 (1983) 1412A-1430A; Fekete, S., et al., Anal. Chem. 93 (2021) 1277-1284). This particular retention behavior has been described as "on/off" or "bind and elute" mechanism, and can be explained as follows: the retention of a large solute is nearly infinite in weak eluent (this corresponds to the "on" or "bind" state, where the proteins species are fully adsorbed at the column inlet), while only a limited increase in eluent strength results in a huge retention decrease (this corresponds to the "off" or "elute" state, as the protein migrates towards the column outlet without any further interactions).

A strategy combining isocratic steps and very short steep gradient segments at protein elution composition, to tune selectivity as desired has been reported (Fekete, S., et al., Anal. Chem. 91 (2019) 12954-12961; Nguyen, J.M., et al., J. Chromatogr. B. 1173 (2021) 122694). Such a strategy has been applied only once in AEX for the separation of empty and full capsids of recombinant adeno-associated viruses (Khatwani, S.L., et al., Mol. Ther. - Methods Clin. Dev. 21 (2021) 548-558).

Based on two initial AEX gradients from 0 % to 60 % B in 15 and 45 minutes, respectively, and the measurement of retention times for the two species, the following gradient was derived: 98 % (v/v) mobile phase A (A) and 2 % (v/v) mobile phase B (B) for 1 min at the beginning of the method. Thereafter a linear change to about 82 % (v/v) A and 18 % (v/v) B in 5 minutes, an isocratic hold for about 4 minutes, and a further linear change to about 40 % (v/v) A and 60 % (v/v) B within 5 minutes.

These conditions were experimentally verified.

Thus, the invention provides a method for separating full and empty recombinant adeno-associated virus particles using an anion exchange chromatography step,
wherein the method comprises a sequence of steps as follows: a) applying a solution comprising empty and/or full rAAV particles to an anion exchange chromatography material inside a chromatography column, b) performing a first isocratic step, c) applying a first linear gradient, d) performing a second isocratic step, and e) applying a second linear gradient,
wherein the empty recombinant adeno-associated virus particles are eluted during the first linear gradient and the full recombinant adeno-associated virus particles are eluted during the second linear gradient.

It has to be pointed out that the second isocratic step has the effect of allowing the entire amount of empty recombinant adeno-associated viral particles to be eluted from the column, prior to changing the conditions to have the full recombinant adeno-associated viral particles eluted from the column.

Different isocratic hold conditions compositions around the derived value were tested, i.e. 17.0 % (v/v), 17.5 % (v/v), 18 % (v/v) and 18.5 % (v/v) B. The corresponding chromatograms are shown as overlay in Figure 6.

It can be seen that a complete separation of full and empty rAAV8 particle peaks was achieved independently of the applied isocratic hold conditions.

To assess the most suitable conditions, the full/empty ratio and the chromatographic resolution were used. When the isocratic step was performed at 17 % (v/v) B or 17.5 % (v/v) B, the peak corresponding to the empty particle (first eluted peak) was broadened compared to the other tested conditions. As a result, the full/empty ratios were too high. When the isocratic step was performed at 18.5 % (v/v) B, the obtained peak shapes were good and the resolution was maximal (Rs of 5.02), but the full/empty ratio was not in agreement with theoretical expectations (only 0.68).

When the isocratic step was performed at 18 % (v/v) B, the resolution was good (Rs of 3.72), no substantial peak tailing/broadening was observed and the full/empty ratio was close to the expected value of 1 (1.14).

In addition to the ionic strength of the second isocratic step, the duration of the second isocratic step can also be used to modify selectivity and resolution. In more detail, the second isocratic step duration affects the retention of the next peak to elute, i.e. the full particles peak. It will have no effect on the previously eluted peak, i.e. the empty particles peak.

To show this, the duration of the second isocratic step was evaluated using durations of 4 minutes, 6 minutes, 8 minutes and 10 minutes. The corresponding chromatograms are shown in Figure 7.

When the duration of the second isocratic step was increased, a decrease in the peak intensity and peak area corresponding to the full rAAV particle was observed, while the peak corresponding to empty rAAV particle was not affected (see Figure 7). Without being bound by this theory, it is assumed that a too long isocratic step results in an incorrect full/empty ratio (too low value).

To show the improvement obtained with the method according to the current invention a comparison was done between the linear gradient elution method (Example 2, Figure 8A), the adjusted linear gradient elution method (Example 3, Figure 8B) and the method according to the current invention (Example 4, Figure 8C).

As can be seen the gain in terms of chromatographic resolution between chromatograms reported in Figures 8A and 8B is modest, while the ratio of full/empty remained comparable. However, the resolution improvement was remarkable when applying the method according to the current invention (Rs of 3.72), while the full/empty ratio remained comparable (1.14).

To check the applicability of the method according to the invention for the characterization of rAAV particles, two different types of samples, including both the full and empty rAAV8 particles, were obtained from two different providers (i.e. Sirion Biotech and Virovek).

With particles obtained from Sirion Biotech, the retention time of empty rAAV particles was slightly larger than for the Virovek supplier. This can be explained by a vector production process that leads to a different amount of negative charges on the capsid, which may induce a somewhat larger retention of the sample. Thus, the separation conditions for full and empty particles for the rAAV from Sirion Biotech were slightly different, and the isocratic step was set at 18.5 % (v/v) of mobile phase B to allow adequate elution of the Sirion empty particles.

Eleven mixtures with both the full and empty particles co-mixed in different percentages (ranging from 0 % to 100 % of full capsids) in PBS buffer were analyzed. Figure 9 shows the corresponding chromatograms of all co-mixtures, with 0:100 and 100:0 empty/full sample corresponding to the pure full and empty rAAV particles, respectively. As shown in the chromatograms, the full capsids sample from Virovek already contains about 10 % of empty particles, while this value drops to only 1-2 % for the Sirion Biotech samples. On the other hand, the empty particle samples from the two providers already contain more than 14 % of full particles. In Figure 9, also a graphic representation of the experimental vs. theoretical percentages of full rAAV particles in the mixtures is provided. The experimental values were exclusively obtained from the peak areas. As can be seen, a linear change in the peak area of both peaks (the R² values were higher than 0.995 for the two different samples), with corresponding changes in percent peak areas of empty and full rAAV particles, is obtained with the method according to the current invention. These observations confirm that the area of each peak was additive, linear and specific to the empty and full rAAV8 particles. This confirms that the method according to the current invention can be used in QC environment, as it is precise, linear, and sufficiently robust.

The examples and figures are provided to aid the understanding of the present invention, the true scope of which is set forth in the appended claims.

### Materials

Bis-Tris propane (BTP, ≥ 99.0%), 2-amino-2-methyl-1,3-propane diol (AMPD, ≥ 99.0%), N-(1,1-dimethyl-2-hydroxyethyl)-3-amino-2-hydroxypropanesulfonic acid (AMPSO, ≥ 99.0%), 2-(cyclohexylamino)ethane sulfonic acid (CHES, BioUltra, ≥ 99.5%), 3-(cyclohexylamino)-2-hydroxy-1-propane sulfonic acid (CAPSO, ≥ 99% anhydrous basis), magnesium chloride hexahydrate (BioXtra, ≥ 99.0%), hydrochloric acid solution (1 N), sodium chloride (BioUltra, for molecular biology, ≥ 99.5%), potassium chloride (BioUltra, for molecular biology, ≥ 99.5%), tetramethyl ammonium chloride (TMAC, LiChropur, ≥ 99.0%) were purchased from Sigma-Aldrich (Buchs, Switzerland). Sodium hydroxide solution (1 N) was obtained from VWR Chemicals. Phosphate-buffered saline (PBS) and poloxamer 188 were obtained from Roche Diagnostics GmbH (Penzberg, Germany). Water was provided by a Milli-Q purification system from Millipore (Bedford, MA, USA).

Samples comprising either full or empty rAAV8 particles were obtained from Virovek (Hayward, CA, USA) at a concentration of 2.00 E+13 vp/mL, and from Sirion Biotech (Graefelfing, Germany) at 5.00 E+13 vp/mL.

Anion exchange chromatography was performed on an ACQUITY UPLC H-Class system (Waters, Milford, MA, USA), equipped with an auto-sampler, including an injection loop of 50 µL, a quaternary solvent delivery pump, and a fluorescence detector (FD). Data were acquired using FLR excitation at 280 nm and emission at 350 nm.

**Table 1. List of chromatographic columns and their properties (chemistry: quaternary ammonium for all columns). PEEK and SS stand for Polyether Ether Ketone and Stainless Steel, respectively.**

| **column** | **name** | **provider** | **stationary phase** | **dimensions [mm]** | **particle size [µm]** |
|---|---|---|---|---|---|
| PP | ProPac SAX-10 | Thermo | Nonporous PEEK | 50 x 4 | 10 |
| PS | ProSwift SAX-1S | Thermo | Monolith PEEK-lined SS | 50 x 4.6 | - |
| AX | Bio-SAX | Agilent | Nonporous PEEK | 50 x 4.6 | 5 |
| QS | TSKgel Q-STAT | Tosoh | Nonporous SS tubing, PEEK frits | 100 x 4.6 | 7 |

The chromatographic columns were kept at room temperature during the analyses. 20 µL of 1.00 E+12 vp/mL samples were injected in all cases, corresponding to a column load of 2.00 E+10 vp. Acquisitions were performed in salt-gradient mode and applied to full, empty, and mix rAAV particle samples. Data acquisition and instrument control were performed by Empower Pro 3 software (Waters).

### Example 1

### Sample preparation

The concentration of rAAV particles in the samples is expressed as the number of viral particles per mL (vp/mL).

Samples were stored at - 80 °C. Prior to analysis, samples were diluted to 1.00 E+12 vp/mL in the appropriate solvent (phosphate buffer solution with 0.001% poloxamer 188 or the respective mobile phase).

For "mix sample" containing both the full and empty rAAV particle, appropriate volumes of the full rAAV particle comprising diluted sample and the empty rAAV particle comprising diluted sample were taken and mixed to obtain the desired ratio of full and empty rAAV particles in the aliquot.

All samples were stored at 4 °C after the sample preparation and analyzed within 72 h after preparation.

### Example 2 - comparative example

### Standard method

Mobile phase A (A) was composed of 65 mM 1,3-bis[tris(hydroxymethyl)methylamino]propane (BTP), 2 mM magnesium chloride hexahydrate in water adjusted to pH 9.0 with 1 M sodium hydroxide solution. Mobile phase B (B) was composed of 65 mM BTP, 2 mM magnesium chloride hexahydrate, 500 mM sodium chloride in water adjusted to pH 9.0 with 1 M sodium hydroxide solution.

The standard method started with 2 % (v/v) B held for 3 minutes. Then, a linear gradient was applied by increasing the fraction of mobile phase B 2 % (v/v) to 56 % (v/v) in 30 min. This was followed by a washing step performed at 100 % (v/v) B for 5 min. and a re-equilibration step of the column at 2 % (v/v) B for 6 min. The column was kept at room temperature and the flow rate was set to a constant value, such as 0.7 mL/min.

### Example 3 - comparative example

### Optimized standard method

Mobile phase A (A) was composed of 65 mM N-(1,1-Dimethyl-2-hydroxyethyl)-3-amino-2-hydroxypropanesulfonic acid (AMPSO), 2 mM magnesium chloride hexahydrate in water adjusted to pH 9.4 with 1 M sodium hydroxide solution. Mobile phase B (B) was composed of 65 mM AMPSO, 2 mM magnesium chloride hexahydrate, 500 mM tetramethyl ammonium chloride in water adjusted to pH 9.4 with 1 M sodium hydroxide solution.

The column was kept at room temperature and the flow rate was set to a constant value, such as 0.3 mL/min. The same linear gradient as described in Example 2 was applied.

### Example 4

### Method according to the invention

Mobile phase A (A) was composed of 65 mM N-(1,1-Dimethyl-2-hydroxyethyl)-3-amino-2-hydroxypropanesulfonic acid (AMPSO), 2 mM magnesium chloride hexahydrate in water adjusted to pH 9.4 with 1 M sodium hydroxide solution. Mobile phase B (B) was composed of 65 mM AMPSO, 2 mM magnesium chloride hexahydrate, 500 mM tetramethyl ammonium chloride in water adjusted to pH 9.4 with 1 M sodium hydroxide solution.

The method according to the invention started by applying a mixture of 98 % (v/v) A and 2 % (v/v) B for 1 minute. Thereafter, the concentration of B was increased from 2 % (v/v) to 18 % (v/v) in 5 min. The concentration of 18 % (v/v) B was held thereafter for 4 min. Thereafter, the concentration of B was increased from 18 % (v/v) to 60 % (v/v) in 5 min., followed by a washing step performed at 100 % (v/v) B for 5 min. and a re- equilibration step at 2 % (v/v) B for 5 min.

The column was kept at room temperature and a constant flow rate was applied, such as 0.3 mL/min.

| **A** | **B** | **step** | **AMPSO** | **MgCl₂** | **TMAC** | **pH** |
|---|---|---|---|---|---|---|
| **[%]** | **[%]** | | **[mM]** | **[mM]** | **[mM]** | |
| 98 | 2 | isocratic 1 | 65 | 2 | 10 | 9.4 |
| 82 | 18 | gradient 1 | 65 | 2 | 90 | 9.4 |
| 82 | 18 | isocratic 2 | 65 | 2 | 90 | 9.4 |
| 40 | 60 | gradient 2 | 65 | 2 | 300 | 9.4 |
| 0 | 100 | wash | 65 | 2 | 500 | 9.4 |

## Claims

1. A method for separating full and empty recombinant adeno-associated virus particles using an anion exchange chromatography step,
wherein the method comprises a sequence of steps as follows:
a) applying a solution comprising empty and full recombinant adeno-associated virus (rAAV) particles to an anion exchange chromatography material inside a chromatography column,
b) performing a first isocratic step,
c) applying a first linear gradient,
d) performing a second isocratic step, and
e) applying a second linear gradient,
wherein the empty recombinant adeno-associated virus particles are eluted during the first linear gradient and the full recombinant adeno-associated virus particles are eluted during the second linear gradient,
wherein the solution comprising empty and full rAAV particles is a phosphate buffered saline solution comprising 0.001 % (w/v) +/- 20% of a non-ionic detergent.

2. The method according to claim 1, wherein the non-ionic detergent is selected from poloxamer 188 and polysorbate 20.

3. The method according to any one of claims 1 to 2, wherein the rAAV particle is of the serotype 8.

4. The method according to any one of claims 1 to 3, wherein the anion exchange chromatography material has a stationary phase consisting of microporous ethylvinylbenzene cross-linked with 55 % divinylbenzene polymer substrate with quaternary ammonium groups providing for the anion exchange functionality.

5. The method according to any one of claims 1 to 4, wherein the anion exchange chromatography column has the dimension of 50 mm +/- 20% length and 4-5 mm +/- 20% diameter, and the anion exchange chromatography material has a particle size of 10 µm +/- 20%.

6. The method according to any one of claims 1 to 5, wherein the method
i) provides baseline separation of full and empty rAAV particles, or
ii) is for quantification of full and empty rAAV particles, or
iii) is a high-throughput method for quantification of full and empty rAAV particles rAAV, or
iv) is a quality control (QC) method for rAAV preparations.

7. The method according to any one of claims 1 to 6, wherein the solutions used in the isocratic steps and linear gradients comprise a buffer substance with a pKa value of 9 +/- 20%.

8. The method according to any one of claims 1 to 7, wherein the solutions used in the isocratic steps and linear gradients comprise as buffer substance N-(1,1-Dimethyl-2-hydroxyethyl)-3-amino-2-hydroxypropane sulfonic acid.

9. The method according to any one of claims 1 to 8, wherein the solutions used in the isocratic steps and linear gradients have a pH value of 9.0 to 9.6.

10. The method according to any one of claims 1 to 9, wherein the solutions used in the isocratic steps and linear gradients comprise as elution salt an organic chloride with a molecular weight below 170 g/mol.

11. The method according to any one of claims 1 to 10, wherein the solutions used in the isocratic steps and linear gradients comprise as elution salt tetramethyl ammonium chloride.

12. The method according to any one of claims 1 to 11, wherein the method is performed at a flow rate of 0.3 mL/min +/- 20%.

13. The method according to any one of claims 1 to 12, wherein the solution applied in the first isocratic step comprises 65 mM +/- 20% of the buffer substance, 10 mM +/- 20% of the elution salt, 2 mM +/- 20% of magnesium chloride and has a pH value of 9.4 +/- 20%,

14. The method according to any one of claims 1 to 13, wherein the solution applied in the second isocratic step comprises 65 mM +/- 20% of the buffer substance, 90 mM +/- 20% of the elution salt, 2 mM +/- 20% of magnesium chloride and has a pH value of 9.4 +/- 20%.

15. The method according to any one of claims 1 to 14, wherein the first isocratic step has a length of 1 minute +/- 20%, the first linear gradient has a length of 5 minute +/- 20%, a second isocratic step has a length of 2 to 5 minutes +/-20% and the second linear gradient has a length of 5 minutes +/- 20%.

## Patentansprüche

1. Verfahren zur Trennung von vollen und leeren rekombinanten Adeno-assoziierten Viruspartikeln unter Verwendung eines Anionenaustauschchromatographieschritts,
wobei das Verfahren eine Sequenz von Schritten wie folgt umfasst:
a) Aufbringen einer Lösung, die leere und volle rekombinante Adeno-assoziierte Virus (rAAV)-Partikel umfasst, auf ein Anionenaustauschchromatographiematerial innerhalb einer Chromatographiesäule,
b) Durchführen eines ersten isokratischen Schritts,
c) Anwenden eines ersten linearen Gradienten,
d) Durchführen eines zweiten isokratischen Schritts und
e) Anwenden eines zweiten linearen Gradienten,
wobei die leeren rekombinanten Adeno-assoziierten Viruspartikel während des ersten linearen Gradienten eluiert werden und die vollen rekombinanten Adeno-assoziierten Viruspartikel während des zweiten linearen Gradienten eluiert werden,
wobei die Lösung, die leere und volle rAAV-Partikel umfasst, eine phosphatgepufferte Salzlösung ist, die 0,001 % (Gew./Vol.) +/- 20 % eines nichtionischen Detergens umfasst.

2. Verfahren nach Anspruch 1, wobei das nichtionische Detergens ausgewählt ist aus Poloxamer 188 und Polysorbat 20.

3. Verfahren nach einem der Ansprüche 1 bis 2, wobei das rAAV-Partikel vom Serotyp 8 ist.

4. Verfahren nach einem der Ansprüche 1 bis 3, wobei das Anionenaustauschchromatographiematerial eine stationäre Phase aufweist, die aus mikroporösem Ethylvinylbenzol besteht, das mit 55 % Divinylbenzolpolymersubstrat mit quartären Ammoniumgruppen vernetzt ist, die die Anionenaustauschfunktionalität bereitstellen.

5. Verfahren nach einem der Ansprüche 1 bis 4, wobei die Anionenaustauschchromatographiesäule die Abmessungen 50 mm +/- 20 % Länge und 4-5 mm +/- 20 % Durchmesser aufweist und das Anionenaustauschchromatographiematerial eine Partikelgröße von 10 µm +/- 20 % aufweist.

6. Verfahren nach einem der Ansprüche 1 bis 5, wobei das Verfahren
i) eine Basislinientrennung von vollen und leeren rAAV-Partikeln bereitstellt oder
ii) zur Quantifizierung von vollen und leeren rAAV-Partikeln dient oder
iii) ein Hochdurchsatzverfahren zur Quantifizierung von vollen und leeren rAAV-Partikeln ist oder
iv) ein Qualitätskontrollverfahren (QC-Verfahren) für rAAV-Präparate ist.

7. Verfahren nach einem der Ansprüche 1 bis 6, wobei die Lösungen, die in den isokratischen Schritten und linearen Gradienten verwendet werden, eine Puffersubstanz mit einem pKa-Wert von 9 +/- 20 % umfassen.

8. Verfahren nach einem der Ansprüche 1 bis 7, wobei die Lösungen, die in den isokratischen Schritten und linearen Gradienten verwendet werden, als Puffersubstanz N-(1,1-Dimethyl-2-hydroxyethyl)-3-amino-2-hydroxypropansulfonsäure umfassen.

9. Verfahren nach einem der Ansprüche 1 bis 8, wobei die Lösungen, die in den isokratischen Schritten und linearen Gradienten verwendet werden, einen pH-Wert von 9,0 bis 9,6 aufweisen.

10. Verfahren nach einem der Ansprüche 1 bis 9, wobei die Lösungen, die in den isokratischen Schritten und linearen Gradienten verwendet werden, als Elutionssalz ein organisches Chlorid mit einem Molekulargewicht unter 170 g/mol umfassen.

11. Verfahren nach einem der Ansprüche 1 bis 10, wobei die Lösungen, die in den isokratischen Schritten und linearen Gradienten verwendet werden, als Elutionssalz Tetramethylammoniumchlorid umfassen.

12. Verfahren nach einem der Ansprüche 1 bis 11, wobei das Verfahren bei einer Flussrate von 0,3 ml/min +/- 20 % durchgeführt wird.

13. Verfahren nach einem der Ansprüche 1 bis 12, wobei die Lösung, die in dem ersten isokratischen Schritt aufgebracht wird, 65 mM +/- 20 % der Puffersubstanz, 10 mM +/-20 % des Elutionssalzes, 2 mM +/- 20 % Magnesiumchlorid umfasst und einen pH-Wert von 9,4 +/- 20 % aufweist.

14. Verfahren nach einem der Ansprüche 1 bis 13, wobei die Lösung, die in dem zweiten isokratischen Schritt aufgebracht wird, 65 mM +/- 20 % der Puffersubstanz, 90 mM +/-20 % des Elutionssalzes, 2 mM +/- 20 % Magnesiumchlorid umfasst und einen pH-Wert von 9,4 +/- 20 % aufweist.

15. Verfahren nach einem der Ansprüche 1 bis 14, wobei der erste isokratische Schritt eine Länge von 1 Minute +/- 20 % aufweist, der erste lineare Gradient eine Länge von 5 Minuten +/- 20 % aufweist, ein zweiter isokratischer Schritt eine Länge von 2 bis 5 Minuten +/- 20 % aufweist und der zweite lineare Gradient eine Länge von 5 Minuten +/-20 % aufweist.

## Revendications

1. Procédé de séparation de particules de virus adéno-associé recombinant pleines et vides en utilisant une étape de chromatographie d'échange d'anions,
dans lequel le procédé comprend une séquence d'étapes comme suit :
a) application d'une solution comprenant des particules de virus adéno-associé recombinant (rAAV) pleines et vides sur un matériau de chromatographie d'échange d'anions à l'intérieur d'une colonne de chromatographie,
b) réalisation d'une première étape isocratique,
c) application d'un premier gradient linéaire,
d) réalisation d'une seconde étape isocratique, et
e) application d'un second gradient linéaire,
dans lequel les particules de virus adéno-associé recombinant vides sont éluées pendant le premier gradient linéaire et les particules de virus adéno-associé recombinant pleines sont éluées pendant le second gradient linéaire,
dans lequel la solution comprenant des particules de rAAV vides et pleines est une solution saline tamponnée au phosphate comprenant 0,001 % (p/v) +/- 20 % d'un détergent non ionique.

2. Procédé selon la revendication 1, dans lequel le détergent non ionique est choisi parmi le poloxamère 188 et le polysorbate 20.

3. Procédé selon l'une quelconque des revendications 1 à 2, dans lequel la particule de rAAV est du sérotype 8.

4. Procédé selon l'une quelconque des revendications 1 à 3, dans lequel le matériau de chromatographie d'échange d'anions a une phase stationnaire constituée d'éthylvinylbenzène microporeux réticulé avec 55 % de substrat polymère de divinylbenzène avec des groupes ammonium quaternaire fournissant la fonctionnalité d'échange d'anions.

5. Procédé selon l'une quelconque des revendications 1 à 4, dans lequel la colonne de chromatographie d'échange d'anions a des dimensions de 50 mm +/- 20 % de longueur et 4 à 5 mm +/- 20 % de diamètre, et le matériau de chromatographie d'échange d'anions a une granulométrie de 10 µm +/- 20 %.

6. Procédé selon l'une quelconque des revendications 1 à 5, dans lequel le procédé
i) fournit une séparation de base des particules de rAAV pleines et vides, ou
ii) est pour la quantification des particules de rAAV pleines et vides, ou
iii) est un procédé à haut débit pour la quantification des particules de rAAV pleines et vides, ou
iv) est un procédé de contrôle de la qualité (QC) pour les préparations de rAAV.

7. Procédé selon l'une quelconque des revendications 1 à 6, dans lequel les solutions utilisées dans les étapes isocratiques et les gradients linéaires comprennent une substance tampon avec une valeur de pKa de 9 +/- 20 %.

8. Procédé selon l'une quelconque des revendications 1 à 7, dans lequel les solutions utilisées dans les étapes isocratiques et les gradients linéaires comprennent en tant que substance tampon de l'acide N-(1,1-diméthyl-2-hydroxyéthyl)-3-amino-2-hydroxypropanesulfonique.

9. Procédé selon l'une quelconque des revendications 1 à 8, dans lequel les solutions utilisées dans les étapes isocratiques et les gradients linéaires ont une valeur de pH de 9,0 à 9,6.

10. Procédé selon l'une quelconque des revendications 1 à 9, dans lequel les solutions utilisées dans les étapes isocratiques et les gradients linéaires comprennent en tant que sel d'élution un chlorure organique avec un poids moléculaire inférieur à 170 g/mol.

11. Procédé selon l'une quelconque des revendications 1 à 10, dans lequel les solutions utilisées dans les étapes isocratiques et les gradients linéaires comprennent en tant que sel d'élution du chlorure de tétraméthylammonium.

12. Procédé selon l'une quelconque des revendications 1 à 11, dans lequel le procédé est réalisé à un débit de 0,3 mL/min +/- 20 %.

13. Procédé selon l'une quelconque des revendications 1 à 12, dans lequel la solution appliquée dans la première étape isocratique comprend 65 mM +/- 20 % de la substance tampon, 10 mM +/- 20 % du sel d'élution, 2 mM +/- 20 % de chlorure de magnésium et a une valeur de pH de 9,4 +/- 20 %.

14. Procédé selon l'une quelconque des revendications 1 à 13, dans lequel la solution appliquée dans la seconde étape isocratique comprend 65 mM +/- 20 % de la substance tampon, 90 mM +/- 20 % du sel d'élution, 2 mM +/- 20 % de chlorure de magnésium et a une valeur de pH de 9,4 +/- 20 %.

15. Procédé selon l'une quelconque des revendications 1 à 14, dans lequel la première étape isocratique a une durée de 1 minute +/- 20 %, le premier gradient linéaire a une durée de 5 minutes +/- 20 %, une seconde étape isocratique a une durée de 2 à 5 minutes +/- 20 % et le second gradient linéaire a une durée de 5 minutes +/- 20 %.
